# EUROPEAN PATENT APPLICATION

(11) **EP 4 603 100 A1**
(43) Date of publication of application: **20.08.2025**
(21) Application number: 23877365.9
(22) Date of filing: 13.10.2023
(51) Int. Cl.: A61K 31/795, A61P 43/00, C08F 20/36

(54) **ANTIFIBROTIC AGENT, PHARMACEUTICAL COMPOSITION FOR TREATING FIBROSIS, AND METHOD FOR INACTIVATING MYOFIBROBLAST**

(30) Priority: 14.10.2022 JP 2022165423
(71) Applicant: Kyushu University, National University Corporation, Nishi-ku Fukuoka-shi Fukuoka 819-0395 (JP); Somar Corporation, Tokyo 104-8109 (JP)
(72) Inventor: ISE Hirohiko, Fukuoka-shi, Fukuoka 819-0395 (JP); MATSUO Saori, Tokyo 104-8109 (JP)
(74) Representative: EP&C
(86) International application number: PCT/JP2023/037231
(87) International publication number: WO 2024/080364

(57) **Abstract**

An antifibrotic agent is provided, including a copolymer having a constitutional unit (a) containing an N-acetylglucosamine group and a constitutional unit (b) containing a structure represented by General Formula (b) (provided that a constitutional unit corresponding to the constitutional unit (a) is excluded). In General Formula (b), Yb represents a divalent linking group including an oxygen atom. Rb represents a hydrogen atom or an organic group. * represents a bonding site.
[Chemical Formula 1]

*-Yb-Rb (b)

## Description

### TECHNICAL FIELD

The present invention relates to an antifibrotic agent, a pharmaceutical composition for treating fibrosis, and a method for inactivating myofibroblasts.

Priority is claimed on Japanese Patent Application No. 2022-165423, filed October 14, 2022, the content of which is incorporated herein by reference.

### BACKGROUND ART

Fibrogenesis of tissue is caused by chronic inflammation associated with repeated damage. Chronic inflammation alters fibroblasts and astrocytes, maintains tissue with myofibroblasts, and activates astrocytes. This activation promotes the enhancement of these cells and the abundant production of the extracellular matrix such as collagen. Chronic inflammatory diseases such as tissue fibrogenesis, cancer, and autoimmunity caused in this manner are caused by continuous inflammation due to intermittent and repetitive tissue damage.

Severe tissue damage caused by repeated tissue damage results in the generation of a large number of cell fragments being released from dying cells. Some intracellular molecules contained in cell debris released from damaged or dead cells play a role in inflammatory cells recognizing tissue damage, and are called damage-associated molecular patterns (DAMPs) (Non-Patent Document 1). DAMPs induce an inflammatory response as a danger signal to protect host tissues from harmful situations such as tissue damage and infection. High-mobility group box 1 (HMGB1), a heat shock protein (HSP), adenosine triphosphate (ATP), and the like have clearly defined functions in cells, but these molecules act as DAMPs in the extracellular space after leaking out from dying cells.

The presence of abundant DAMPs after severe tissue damage induces the recruitment and activation of immune cells that secrete inflammatory cytokines and profibrogenic cytokines (Non-Patent Document 2). These cytokines then induce differentiation of astrocytes and fibroblasts into the activated astrocytes and myofibroblasts, and promote hyperplasia or fibrogenesis during tissue remodeling (Non-Patent Document 3). As described above, tissue fibrogenesis is caused by chronic inflammation associated with repeated damage. In chronic inflammation, the excretion of parenchymal cells along with the abundant deposition of collagen finally causes dysfunction of the fibrous tissue.

To recover from tissue fibrogenesis, it is necessary to selectively target myofibroblasts or activated astrocytes and suppress the activation of each cell. For example, to inhibit the expression level of collagen 1A1 and/or α-smooth muscle actin (αSMA) in human lung fibroblasts exposed to SERPINE2, a method of administering an antagonist of SERPINE2 to human lung fibroblasts has been proposed (Patent Document 1). In addition, as a therapeutic drug targeting chronic inflammation or a fibrotic site, for example, a therapeutic drug for fibrosis (Patent Document 2) has been proposed, which contains a polypeptide consisting of a specific amino acid sequence or an amino acid sequence having 85% or more identity to the specific amino acid sequence, and at least one selected from the group consisting of COL1A1, COL1A2, and αSMA, .

### Citation List

### Patent Documents

Patent Document 1: Published Japanese Translation No. 2012-509941 of the PCT International Publication
Patent Document 2: Japanese Unexamined Patent Application, First Publication No. 2022-66026

### Non-Patent Documents

Non-Patent Document 1: Bianchi ME., "DAMPs, PAMPs and alarmins: all we need to know about danger.", J. Leukoc. Biol., Vol. 81, Issue 1, pp. 1-5, 2007. doi: 10.1189/jlb.0306164. Epub 2006 Oct 10. PMID: 17032697.
Non-Patent Document 2: Bolourani, S., "The interplay of DAMPs, TLR4, and proinflammatory cytokines in pulmonary fibrosis.", J. Mol. Med. (Berl)., Vol. 99, Issue 10, pp. 1373-1384, 2021.
Non-Patent Document 3: An, P. et al., "Hepatocyte mitochondria-derived danger signals directly activate hepatic stellate cells and drive progression of liver fibrosis.", Nat. Commun., Vol. 11, Article No. 2362, 2020.

### SUMMARY OF INVENTION

### Technical Problem

Although there are the findings described above, there is a demand for a more effective antifibrotic agent which can induce the inactivation of myofibroblasts and can suppress fibrogenesis.

Therefore, an object of the present invention is to provide an antifibrotic agent capable of inducing the inactivation of myofibroblasts, a pharmaceutical composition for treating fibrosis, containing the antifibrotic agent, and a method of inactivating the myofibroblasts.

### Solution to Problem

As a result of intensive studies, the present inventors have found that a copolymer having a specific constitutional unit containing N-acetylglucosamine and the other constitutional units targets myofibroblasts and suppresses the activation thereof, thereby completing the present invention.

The present invention includes the following aspects.
[1] An antifibrotic agent including a copolymer having a constitutional unit (a) containing an N-acetylglucosamine group and a constitutional unit (b) containing a structure represented by General Formula (b) (provided that a constitutional unit corresponding to the constitutional unit (a) is excluded).
   [Chemical Formula 1]

   *-Yb-Rb (b)

   [In the formula, Yb represents a divalent linking group including an oxygen atom, and Rb represents a hydrogen atom or an organic group. * represents a bonding site.]
[2] The antifibrotic agent according to [1], in which the constitutional unit (b) is a constitutional unit represented by General Formula (b-1). 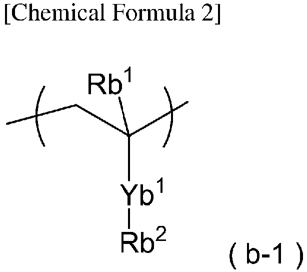 [In the formula, Rb¹ represents a hydrogen atom or an organic group, Yb1 represents -C(=O)-O- or -C(=O)-NH-, and Rb² represents a hydrogen atom or an organic group.]
[3] The antifibrotic agent according to [1], in which the copolymer is a compound represented by General Formula (b-2). 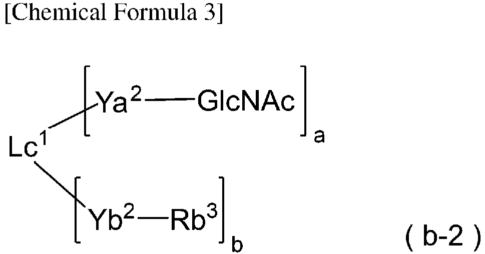 [In the formula, Lc¹ represents an (a + b)-valent linking group, Ya² and Yb² each independently represents a divalent linking group including an oxygen atom, GlcNAC represents an N-acetylglucosamine group, Rb³ represents an organic group, a and b each independently represents an integer of 1 or more as long as an atomic valence is allowed. In a case where a is an integer of 2 or more, two or more Ya²'s may be the same or different from each other. In a case where b is an integer of 2 or more, 2 or more Yb²'s may be the same as or different from each other and 2 or more Rb³'s may be the same as or different from each other.]
[4] The antifibrotic agent according to any one of [1] to [3], in which a molar ratio of the constitutional unit (a) to the constitutional unit (b) is 10:1 to 1:20.
[5] The antifibrotic agent according to [2], in which Rb² in General Formula (b-1) includes at least one structure selected from the group consisting of an ether bond, an ester bond, a urethane bond, and an amide bond.
[6] The antifibrotic agent according to any one of [1] to [5], in which the constitutional unit (a) is a constitutional unit containing a structure represented by Formula (a1-1-1). 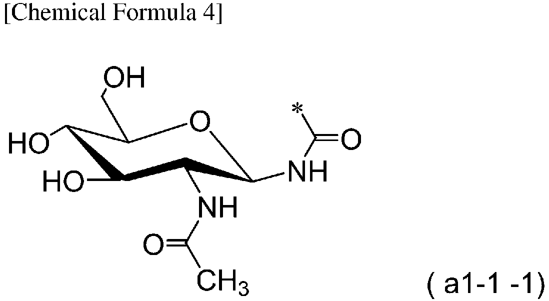 [In the formulae, * represents a bonding site.]
[7] The antifibrotic agent according to [1] or [2], in which the constitutional unit (a) is a constitutional unit represented by Formula (a-1-1). 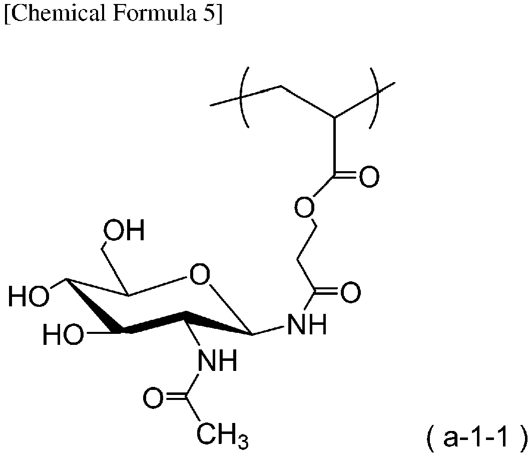
[8] The antifibrotic agent according to [2], in which the constitutional unit (b) is a constitutional unit derived from acrylamide or a compound represented by General Formula (b1-1). 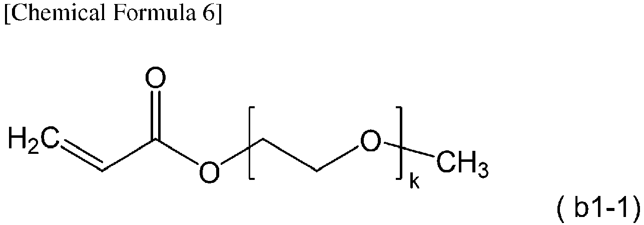 [In the formula, k represents an integer of 1 to 12.]
[9] The antifibrotic agent according to any one of [1] to [8], in which the copolymer is a copolymer represented by any of General Formulae (C-1) to (C-5). 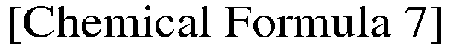 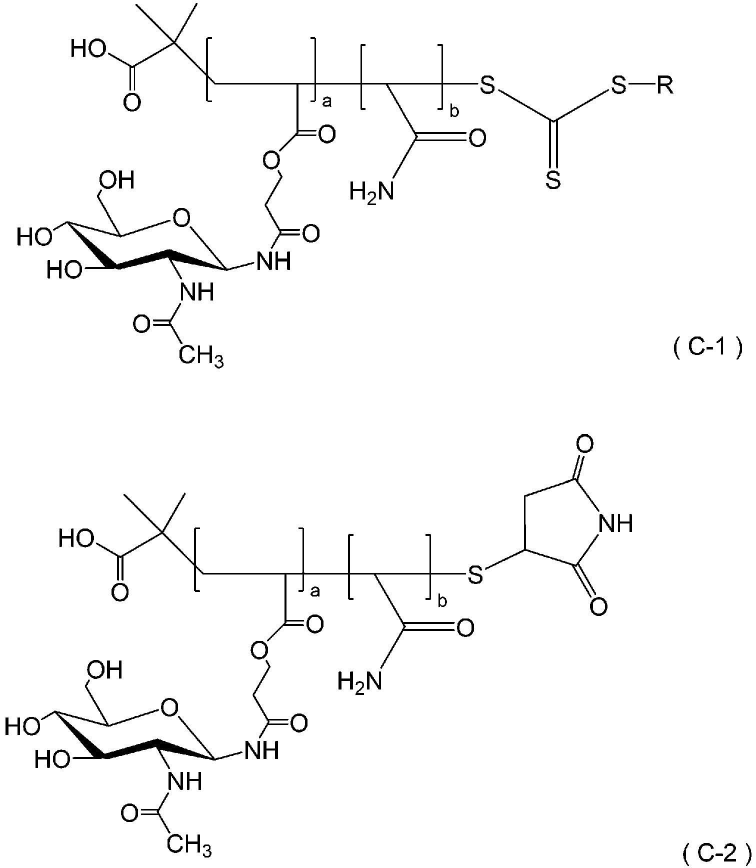 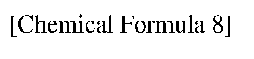 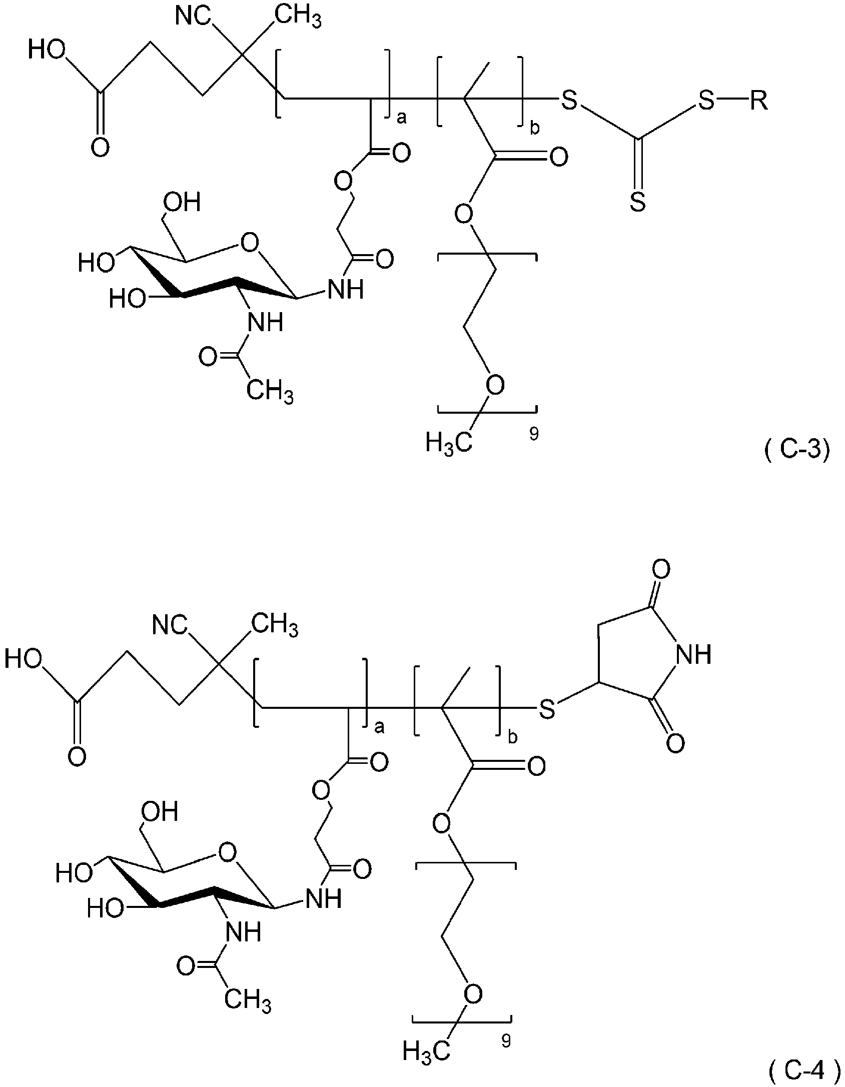 [In the formula, R represents an alkyl group having 1 to 18 carbon atoms, and a and b each independently represents an integer of 1 to 30.] 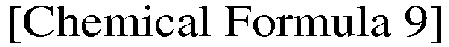 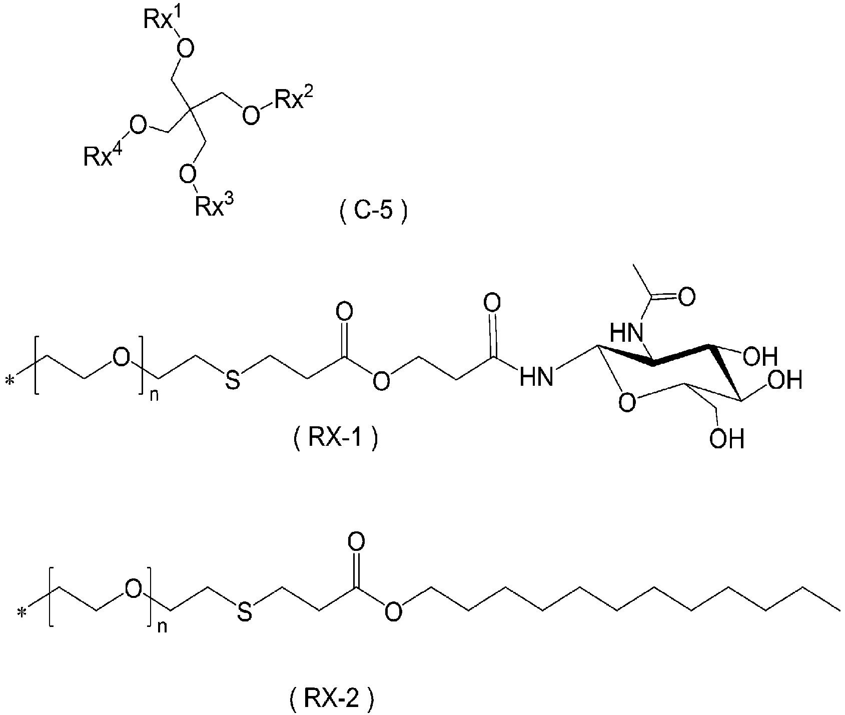 [In the formula, Rx¹ to Rx⁴ each independently represents a group represented by Formula (RX-1) or (RX-2), provided that at least one of Rx¹ to Rx⁴ is a group represented by Formula (RX-1). n represents an integer of 1 or more. * represents a bonding site.]
[10] The antifibrotic agent according to any one of [1] to [9], in which a weight-average molecular weight of the copolymer is within a range of 2,000 to 20,000.
[11] The antifibrotic agent according to any one of [1] to [10], in which a number-average molecular weight of the copolymer is within a range of 2,000 to 15,000.
[12] The antifibrotic agent according to any one of [1] to [11], in which a ratio (Mw/Mn) of a weight-average molecular weight (Mw) to a number-average molecular weight (Mn) of the copolymer is within a range of 1.0 to 1.7.
[13] A pharmaceutical composition for treating fibrosis, including the antifibrotic agent according to any one of [1] to [12] as an active ingredient.
[14] A method of inactivating myofibroblasts, the method including binding the antifibrotic agent according to any one of [1] to [12] to myofibroblasts.

### Advantageous Effects of Invention

According to the present invention, it is possible to provide an antifibrotic agent capable of inducing the inactivation of myofibroblasts, a pharmaceutical composition for treating fibrosis, containing the antifibrotic agent, and a method of inactivating the myofibroblasts.

### BRIEF DESCRIPTION OF DRAWINGS

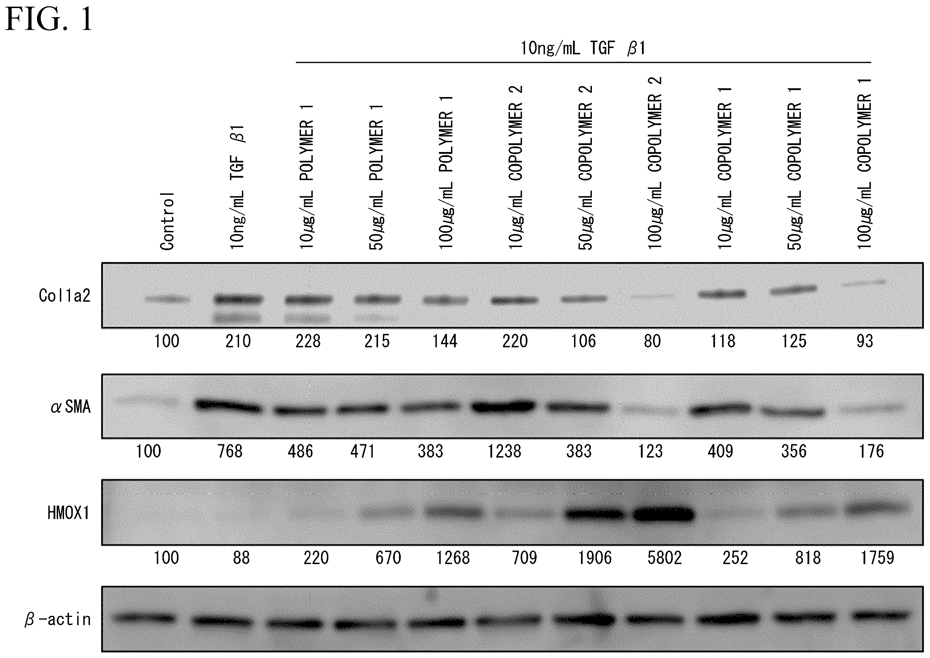

[FIG. 1] A view showing the results of Western blotting in Example 1. Human dermal fibroblasts are cultured for 48 hours in the presence of 10 ng/mL TGF-β1 to obtain myofibroblasts, the test compound (Copolymer 1, Copolymer 2, or Polymer 1) is added to the myofibroblasts (10 µg/mL, 50 µg/mL, or 100 µg/mL), and the mixture is cultured for 48 hours. Thereafter, the expression levels of αSMA, collagen (col1a2), heme oxygenase 1 (HMOX1), and β-actin are confirmed by Western blotting. "Control" indicates the results of culturing human dermal fibroblasts without adding anything, and "10 ng/mL TGF β1" indicates the results of culturing myofibroblasts without adding the test compound.

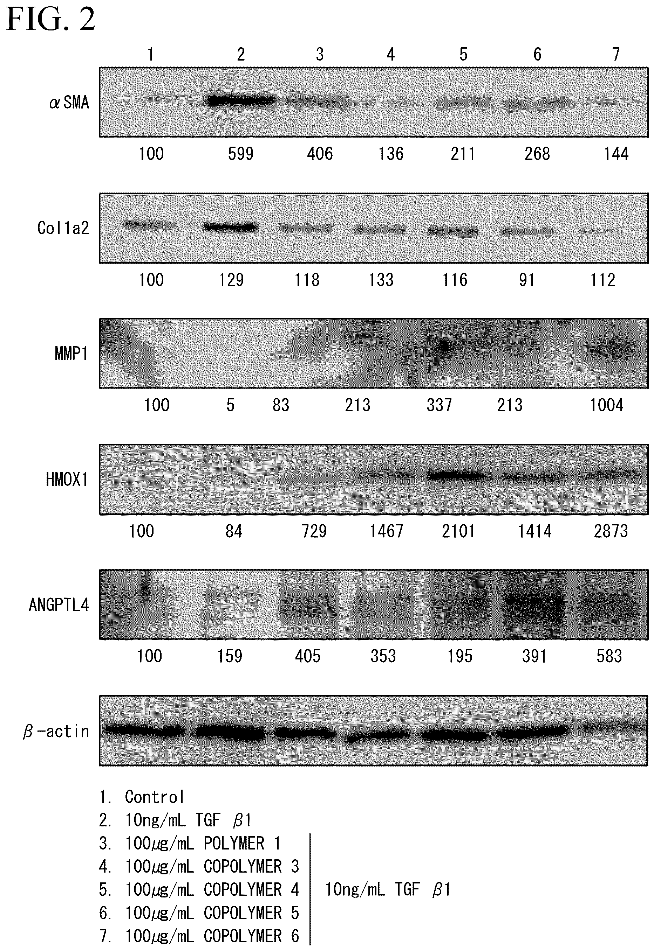

[FIG. 2] A view showing the results of Western blotting in Example 2. Human dermal fibroblasts are cultured for 48 hours in the presence of 10 ng/mL TGF-β1 to obtain myofibroblasts, the test compound (Copolymer 3, Copolymer 4, Copolymer 5, Copolymer 6, or Polymer 1) is added to the myofibroblasts (100 µg/mL), and the mixture is cultured for 48 hours. Thereafter, the expression levels of αSMA, collagen (col1a2), matrix metalloproteinase 1 (MMP1), HMOX1, angiopoietin-like 4 (ANGPTLA), and β-actin are confirmed by Western blotting. "Control" indicates the results of culturing human dermal fibroblasts without adding anything, and "10 ng/mL TGF β1" indicates the results of culturing myofibroblasts without adding the test compound.

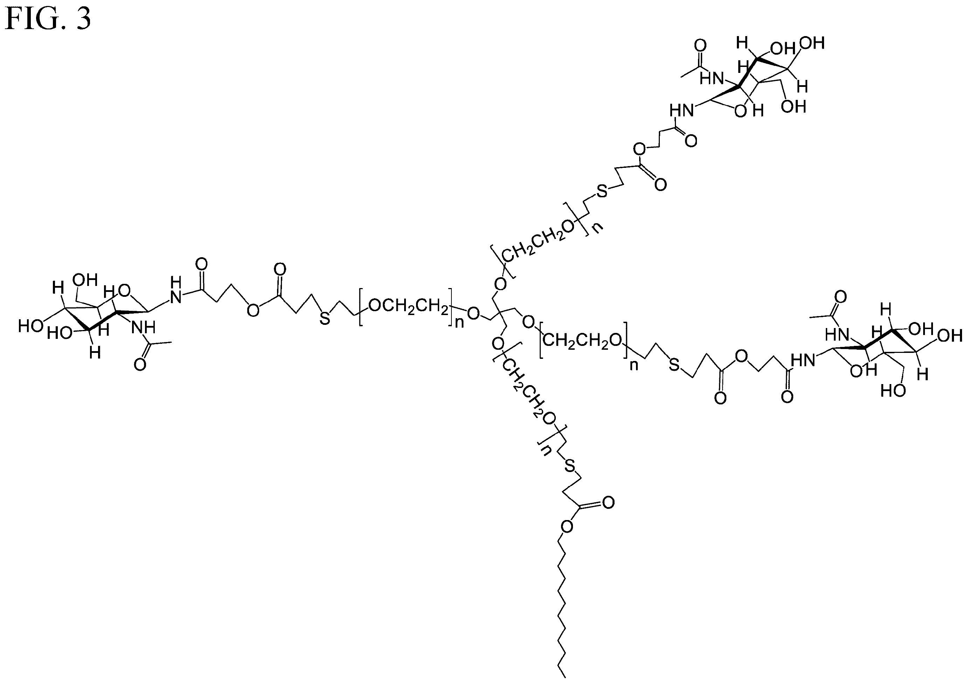

[FIG. 3] A structure of Copolymer 8 used in Example 4.

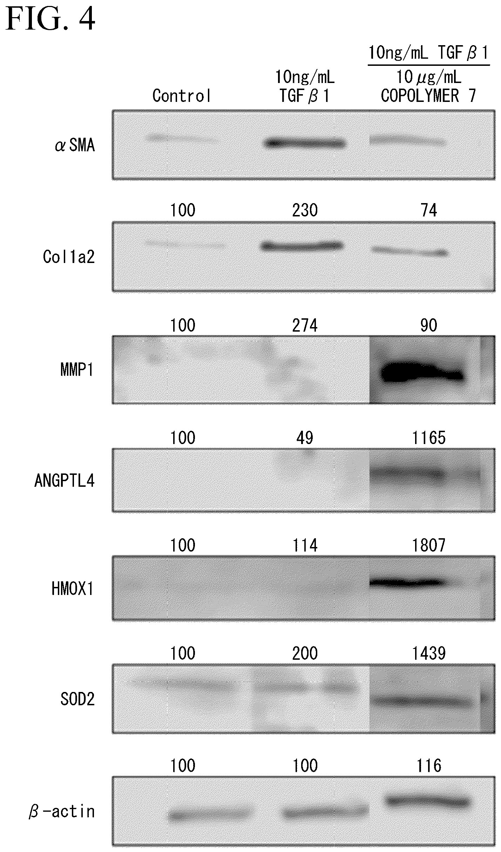

[FIG. 4] A view showing the results of Western blotting in Example 3. Human dermal fibroblasts are cultured for 48 hours in the presence of 10 ng/mL TGF-β1 to obtain myofibroblasts, the test compound (Copolymer 7) is added to the myofibroblasts (10 µg/mL), and the mixture is cultured for 48 hours. Thereafter, the expression levels of αSMA, collagen (col1a2), matrix metalloproteinase 1 (MMP1), HMOX1, ANGPTL4, SOD2, and β-actin are confirmed by Western blotting. "Control" indicates the results of culturing human dermal fibroblasts without adding anything, and "10 ng/mL TGF β1" indicates the results of culturing myofibroblasts without adding the test compound.

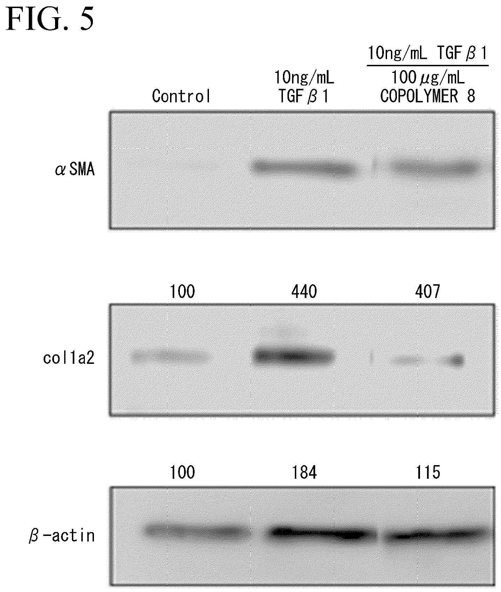

[FIG. 5] A view showing the results of Western blotting in Example 4. Human dermal fibroblasts are cultured for 48 hours in the presence of 10 ng/mL TGF-β1 to obtain myofibroblasts, the test compound (Copolymer 8) is added to the myofibroblasts (100 µg/mL), and the mixture is cultured for 48 hours. Thereafter, the expression levels of αSMA, collagen (col1a2), and β-actin are confirmed by Western blotting. "Control" indicates the results of culturing human dermal fibroblasts without adding anything, and "10 ng/mL TGF β1" indicates the results of culturing myofibroblasts without adding the test compound.

### DESCRIPTION OF EMBODIMENTS

### (Antifibrotic agent)

The antifibrotic agent of the present embodiment contains a copolymer (hereinafter, also referred to as a "copolymer C") having a constitutional unit (a) containing an N-acetylglucosamine group and a constitutional unit (b) containing a structure represented by General Formula (b) (provided that a constitutional unit corresponding to the constitutional unit (a) is excluded).

[Chemical Formula 10]

*-Yb-Rb (b)

[In the formula, Yb represents a divalent linking group including an oxygen atom, and Rb represents a hydrogen atom or an organic group.]

In one embodiment, the constitutional unit (b) is a constitutional unit represented by General Formula (b-1). 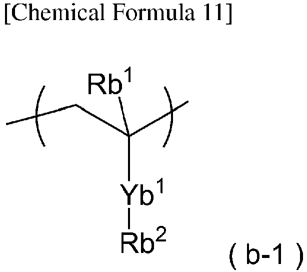 [In the formula, Rb¹ represents a hydrogen atom or an organic group, Yb¹ represents C(=O)-O- or -C(=O)-NH-, and Rb² represents a hydrogen atom or an organic group.]

In one embodiment, a copolymer (C) is a compound represented by General Formula (b-2). 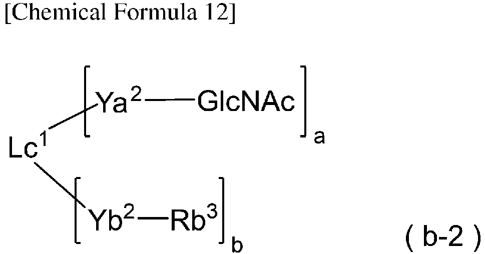 [In the formula, Lc¹ represents an (a + b)-valent linking group, Ya² and Yb² each independently represents a divalent linking group including an oxygen atom, GlcNAC represents an N-acetylglucosamine group, Rb³ represents an organic group, a and b represent an integer of 1 or more as long as an atomic valence is allowed. In a case where a is an integer of 2 or more, two or more Ya²'s may be the same or different from each other. In a case where b is an integer of 2 or more, 2 or more Yb²'s may be the same as or different from each other and 2 or more Rb³'s may be the same as or different from each other.]

### <Copolymer (C)>

A copolymer (C) has a constitutional unit (a) and a constitutional unit (b).

### <<Constitutional unit (a)>>

The constitutional unit (a) is a constitutional unit including an N-acetylglucosamine group. Examples of the constitutional unit (a) include a constitutional unit containing N-acetylglucosamine, chitobiose, chitotriose, chitotetraose, chitopentaose, chitohexaose, or the like. Specific examples of the constitutional unit (a) include a constitutional unit derived from a monomer containing an N-acetylglucosamine group, a constitutional unit derived from a monomer containing a chitopolyose group in which 2 to 6 N-acetylglucosamine rings are bonded.

The N-acetylglucosamine group is a group represented by Formula (a1-1). In the following formula, * represents a bonding site.

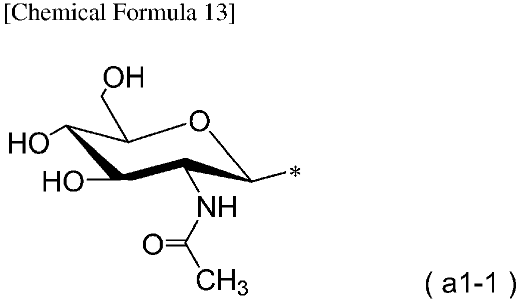

The constitutional unit (a) preferably includes a structure represented by Formula (a1-1-1). In the following formula, * represents a bonding site.

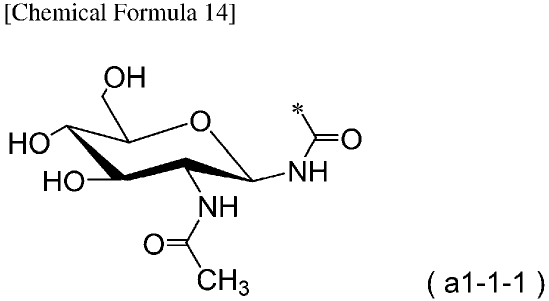

The constitutional unit (a) more preferably includes a structure represented by Formula (a1-1-11). In the following formula, * represents a bonding site.

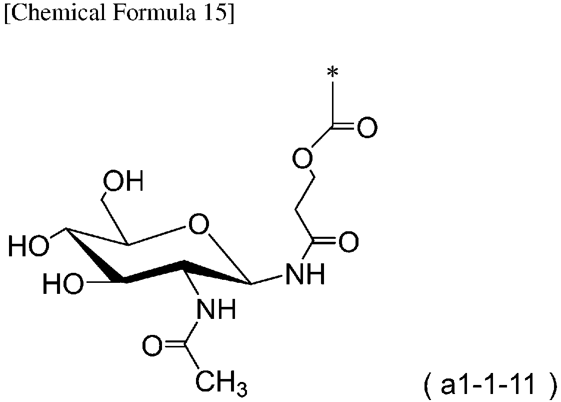

Examples of the constitutional unit (a) include a constitutional unit represented by General Formula (a-1). 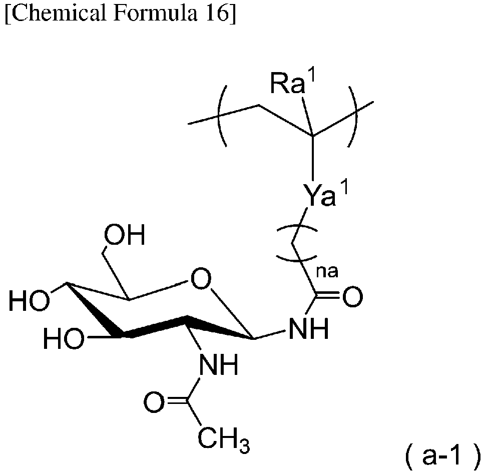 [In the formula, Ra¹ represents a hydrogen atom or an organic group, Ya¹ represents -C(=O)-O- or -C(=O)-NH-, and na represents an integer of 1 to 6.]

Examples of the organic group in Ra1 in Formula (a-1) include a hydrocarbon group which may have a substituent. The hydrocarbon group in Ra1 preferably has 1 to 12 carbon atoms, more preferably has 1 to 10 carbon atoms, still more preferably has 1 to 6 carbon atoms, even still more preferably has 1 to 4 carbon atoms, and particularly preferably has 1 to 3 carbon atoms. The hydrocarbon group which may have a substituent may be an aromatic hydrocarbon group which may have a substituent or an aliphatic hydrocarbon group which may have a substituent, but is preferably an aliphatic hydrocarbon group which may have a substituent. The hydrocarbon group which may have a substituent may be saturated or unsaturated, but is preferably saturated.

Examples of the aliphatic hydrocarbon group in Ra¹ include an alkyl group having 1 to 12 carbon atoms, which may have a substituent. The above-described alkyl group may be linear or branched, but is preferably linear.

The aliphatic hydrocarbon group in Ra¹ may or may not have a substituent. Examples of the substituent which may be contained in the aliphatic hydrocarbon group in Ra¹ include a carboxy group, an amino group, a hydroxy group, an alkoxy group, and the like.

Ra¹ is preferably a hydrogen atom or an alkyl group having 1 to 3 carbon atoms, and more preferably a hydrogen atom or a methyl group.

In Formula (a-1), in a case where -C(=O)-O- and -C(=O)-NH- in Ya¹ are each represented by *-C(=O)-O-** and *-C(=O)-NH-**, * is bonded to a carbon atom to which Ra¹ in Formula (a-1) is bonded, and ** is bonded to -(CH₂)ₙₐ- in Formula (a-1).

In Formula (a-1), na is preferably an integer of 1 to 4, more preferably an integer of 1 to 3, and still more preferably 1 or 2.

The constitutional unit (a) is preferably a constitutional unit represented by General Formula (a-1-1).

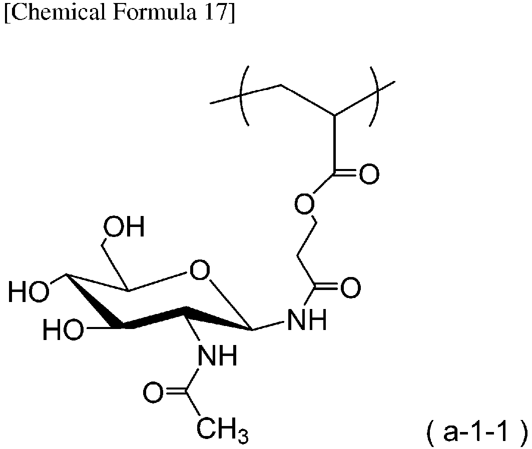

In a case where the copolymer (C) is a compound represented by General Formula (b-2), a structure represented by -Ya²-GlcNAc in General Formula (b-2) is the constitutional unit (a).

In General Formula (b-2), Ya² is a divalent linking group including an oxygen atom. Examples of Ya² include a divalent linking group including at least one selected from the group consisting of an ether bond, an ester bond, an amide bond, a urethane bond, and a thioester bond. Examples of Ya² include a divalent linking group which is a combination of at least one selected from the group consisting of an ether bond, an ester bond, an amide bond, a urethane bond, and a thioester bond, and an alkylene group.

Ya² may include at least one selected from the group consisting of an alkyl group having 1 to 10 carbon atoms, an alkoxyalkylene group having 1 to 10 carbon atoms, a polyether group, a polyester group, a polyurethane group, and a polyamino acid group. The polyether group is preferably a polyethylene glycol group.

Ya² is preferably a divalent linking group which is a combination of at least one selected from the group consisting of an alkyl group having 1 to 10 carbon atoms, an alkoxyalkylene group, and a polyethylene glycol group, and at least one selected from the group consisting of an ether bond, an ester bond, an amide bond, a urethane bond, and a thioester bond.

In General Formula (b-2), GlcNAc is an N-acetylglucosamine group and is a group represented by Formula (a1-1).

Specific examples of the constitutional unit (a) in the copolymer (C) represented by General Formula (b-2) include a constitutional unit having a structure represented by Formula (RX-1). In the following formula, * represents a bonding site bonded to Lc1 in General Formula (b-2). 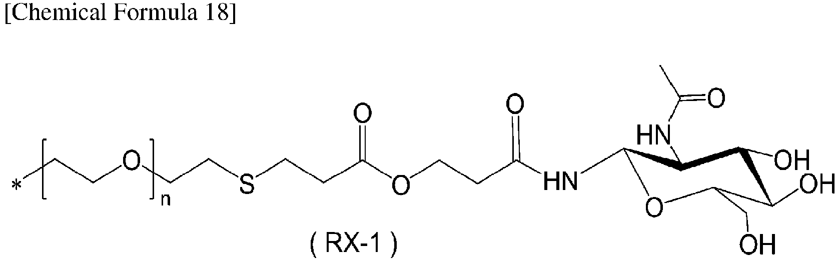 [In the formula, n represents an integer of 1 or more.]

In Formula (RX-1), examples of n include an integer of 1 to 30, and n is preferably an integer of 10 to 30.

Method for manufacturing compound from which constitutional unit (a) is derived:
The compound from which the constitutional unit (a) is derived can be manufactured by a known method. The monomer from which the constitutional unit (a) is derived can be synthesized, for example, by bonding an α,β-unsaturated carboxylic acid to a reducing terminal of N-acetylglucosamine.

Specific examples of the α,β-unsaturated carboxylic acid include acrylic acid, methacrylic acid, acryloxypropionic acid, citraconic acid, itaconic acid, crotonic acid, maleic acid, and maleic acid anhydride. Among these, acrylic acid and methacrylic acid are preferable since they have good copolymerizability with other constitutional units. The α,β-unsaturated carboxylic acid may be in a form of a salt. Examples of the salt of the α,β-unsaturated carboxylic acid include a metal salt such as a sodium salt or a potassium salt of the α,β-unsaturated carboxylic acid monomer, and an ammonium salt of the β-unsaturated carboxylic acid.

Specific examples of the method for manufacturing a compound from which the constitutional unit (a) is derived include a method of obtaining an acrylic monomer into which an N-acetylglucosamine group is introduced by performing a condensation reaction of an α,β-unsaturated carboxylic acid and aminated N-acetylglucosamine using a condensing agent such as 4-(4,6-dimethoxy-1,3,5-triazin-2-yl)-4-methylmorpholinium chloride (DMT-MM). For example, a monomer from which the constitutional unit (a) is derived can be manufactured by mixing aminated N-acetylglucosamine with an α,β-unsaturated carboxylic acid at a molar ratio of 1:1 and performing a condensation reaction in a solvent such as dimethyl sulfoxide (DMSO) or water in the presence of a condensing agent such as DMT-MM.

The method of aminating N-acetylglucosamine is not particularly limited, and examples thereof include a method of binding an amino group of a compound having an amino group to a reducing terminal of N-acetylglucosamine by a reductive amination method. Alternatively, a compound having an amino group may be bonded by a coupling method using a condensing agent such as 1-ethyl-3-(3-dimethylaminopropyl)carbodiimide (EDC) to N-acetylglucosamine in which a hydroxy group of is substituted with a carboxy group.

The constitutional unit (a) may be used alone or in combination of two or more types thereof.

### <<Constitutional unit (b)>>

The constitutional unit (b) is a constitutional unit including a structure represented by General Formula (b).

In General Formula (b), Yb represents a divalent linking group including an oxygen atom. Examples of Yb include a divalent linking group including at least one selected from the group consisting of an ether bond, an ester bond, an amide bond, a urethane bond, and a thioester bond. Yb may be a combination of at least one selected from the group consisting of an ether bond, an ester bond, an amide bond, a urethane bond, and a thioester bond, and an alkylene group.

In General Formula (b), Rb represents a hydrogen atom or an organic group (provided that a group including an N-acetylglucosamine group is excluded). The organic group in Rb is not particularly limited. Examples of the organic group in Rb include a hydrocarbon group which may have a substituent. The hydrocarbon group may be an aromatic hydrocarbon group or an aliphatic hydrocarbon group. The aliphatic hydrocarbon group may be saturated or unsaturated, but is preferably saturated. Examples of the organic group in Rb include a hydrocarbon group having 1 to 20 carbon atoms. The hydrocarbon group may or may not have a substituent. Examples of the substituent include a carboxy group, an amino group, a hydroxy group, an alkoxy group, and the like.

Examples of the constitutional unit (b) include a constitutional unit represented by General Formula (b-1).

In Formula (b-1), examples of the organic group in Rb¹ include a hydrocarbon group which may have a substituent. The hydrocarbon group in Rb¹ preferably has 1 to 12 carbon atoms, more preferably has 1 to 10 carbon atoms, still more preferably has 1 to 6 carbon atoms, even still more preferably has 1 to 4 carbon atoms, and particularly preferably has 1 to 3 carbon atoms. The hydrocarbon group which may have a substituent may be an aromatic hydrocarbon group which may have a substituent or an aliphatic hydrocarbon group which may have a substituent, but is preferably an aliphatic hydrocarbon group which may have a substituent. The hydrocarbon group which may have a substituent may be saturated or unsaturated, but is preferably saturated. As the organic group in Rb1, the organic group containing an N-acetylglucosamine group is excluded.

Examples of the aliphatic hydrocarbon group in Rb¹ include an alkyl group having 1 to 12 carbon atoms, which may have a substituent. The above-described alkyl group may be linear or branched, but is preferably linear.

The aliphatic hydrocarbon group in Rb¹ may or may not have a substituent. Examples of the substituent which may be contained in the aliphatic hydrocarbon group in Rb¹ include a carboxy group, an amino group, a hydroxy group, an alkoxy group, and the like.

Rb¹ is preferably a hydrogen atom or an alkyl group having 1 to 3 carbon atoms, and more preferably a hydrogen atom or a methyl group.

In Formula (b-1), in a case where -C(=O)-O- and -C(=O)-NH- in Yb¹ are each represented by *-C(=O)-O-** and *-C(=O)-NH-**, * is bonded to a carbon atom to which Rb¹ in Formula (b-1) is bonded, and ** is bonded to Rb² in Formula (b-1).

In Formula (b-1), examples of the organic group in Rb² include a hydrocarbon group which may have a substituent. The hydrocarbon group in Rb² preferably has 1 to 20 carbon atoms, more preferably has 1 to 16 carbon atoms, still more preferably has 1 to 12 carbon atoms, and particularly preferably has 1 to 10 carbon atoms. The hydrocarbon group which may have a substituent may be an aromatic hydrocarbon group which may have a substituent or an aliphatic hydrocarbon group which may have a substituent, but is preferably an aliphatic hydrocarbon group which may have a substituent. The hydrocarbon group which may have a substituent may be saturated or unsaturated, but is preferably saturated. As the organic group in Rb², the organic group containing an N-acetylglucosamine group is excluded.

Examples of the aliphatic hydrocarbon group in Rb² include an alkyl group having 1 to 20 carbon atoms, which may have a substituent. The above-described alkyl group may be linear or branched, but is preferably linear.

The aliphatic hydrocarbon group in Rb² may or may not have a substituent. Examples of the substituent which may be contained in the aliphatic hydrocarbon group in Rb² include a carboxy group, an amino group, a hydroxyl group, and the like. The substituent in Rb² may be a substitute for substituting a methylene group (-CH2-) constituting a hydrocarbon chain. Examples of the substituent for substituting the methylene group include an ester bond (-C(=O)-O-), an ether bond (-O-), an amide bond (-C(=O)-NH-), a urethane bond (-NH-C(=O)-O-), a thioester bond (-C(=O)-S-), and the like. Rb² may be a group including at least one structure selected from the group consisting of an ether bond, an ester bond, a urethane bond, and an amide bond.

Specific examples of the organic group in Rb² include an alkyl group, an alkoxyalkylene group, a polyether group, a polyester group, a polyurethane group, and a polyamino acid group.

Rb² is preferably a hydrogen atom, an alkyl group having 1 to 10 carbon atoms, an alkoxyalkylene group, or a polyethylene glycol group.

Specific examples of the constitutional unit (b) are shown below, but it is not limited thereto. nb in Formula (b-1-2) is preferably an integer of 1 to 10 and more preferably an integer of 1 to 9. nb is, for example, 9. 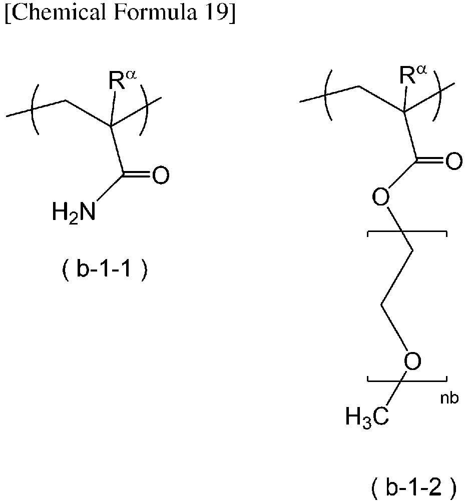 [In the formula, nb represents an integer of 1 to 12. R^{α} represents a hydrogen atom or an alkyl group having 1 to 3 carbon atoms.]

In a case where the copolymer (C) is a compound represented by General Formula (b-2), a structure represented by -Yb²-Rb³ in General Formula (b-2) is the constitutional unit (b).

In General Formula (b-2), Yb² is a divalent linking group including an oxygen atom. Examples of the divalent linking group including an oxygen atom in Yb² include the same groups as those in the above-described Ya².

In General Formula (b-2), Rb³ represents an organic group. Examples of the organic group include a hydrocarbon group which may have a substituent. The hydrocarbon group preferably has 1 to 20 carbon atoms, more preferably has 1 to 18 carbon atoms, still more preferably has 1 to 16 carbon atoms, and particularly preferably has 1 to 12 carbon atoms. The hydrocarbon group which may have a substituent may be an aromatic hydrocarbon group which may have a substituent or an aliphatic hydrocarbon group which may have a substituent, but is preferably an aliphatic hydrocarbon group which may have a substituent. The hydrocarbon group which may have a substituent may be saturated or unsaturated, but is preferably saturated. As the organic group in Rb³, the organic group containing an N-acetylglucosamine group is excluded.

Examples of the aliphatic hydrocarbon group in Rb³ include an alkyl group having 1 to 20 carbon atoms, which may have a substituent. The above-described alkyl group may be linear or branched, but is preferably linear.

The aliphatic hydrocarbon group in Rb³ may or may not have a substituent. Examples of the substituent which may be contained in the aliphatic hydrocarbon group in Rb² include a carboxy group, an amino group, a hydroxyl group, and the like.

Specific examples of the constitutional unit (b) in the copolymer (C) represented by General Formula (b-2) include a constitutional unit having a structure represented by Formula (RX-2-1). In the following formula, * represents a bonding site bonded to Lc1 in General Formula (b-2). 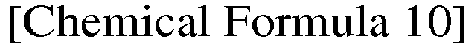 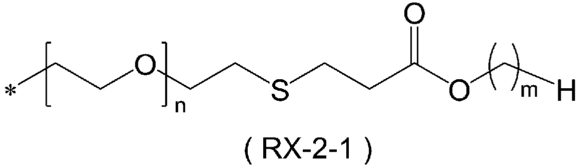 [In the formula, m and n each independently represents an integer of 1 or more.]

In Formula (RX-2-1), m is preferably an integer of 1 to 20, more preferably an integer of 1 to 16, still more preferably an integer of 1 to 14, and particularly preferably an integer of 1 to 12. Specific examples of m include 12.

In Formula (RX-2-1), examples of n include an integer of 1 to 30, and n is preferably an integer of 10 to 30.

Compound from which constitutional unit (b) is derived:
Specific examples of the compound from which the constitutional unit (b) is derived include an α,β-unsaturated carboxylic acid monomer; a monomer in which an acryloyl group, a methacryloyl group, or the like is added to one terminal of a polyether, a polyurethane, a polyamino acid, or a polyester; acrylamide; an ester of an alkoxy alcohol and acrylic acid or methacrylic acid; and the like.

Specific examples of the α,β-unsaturated carboxylic acid monomer include acrylic acid, methacrylic acid, acryloxypropionic acid, citraconic acid, itaconic acid, crotonic acid, maleic acid, and maleic acid anhydride. Among these, acrylic acid and methacrylic acid are preferable since they have good copolymerizability with other constitutional units.

Examples of the monomer in which an acryloyl group, a methacryloyl group, or the like is added to one terminal of polyether, polyurethane, polyamino acid, or polyester include a condensate of the compound and acrylic acid or methacrylic acid. Among these, polyethylene glycol monomethyl ether methacrylate, polyethylene glycol monomethyl ether acrylate, or the like is preferable since it has good copolymerizability with other constitutional units and good arrangement between the constitutional units (a).

Examples of the ester of an alkoxy alcohol with an acrylic acid or a methacrylic acid include 2-methoxyethyl acrylate, 2-methoxyethyl methacrylate, and the like.

As the compound from which the constitutional unit (b) is derived, acrylamide or a compound represented by General Formula (b1-1) is preferable. In Formula (b1-1), k is preferably an integer of 1 to 10 and more preferably an integer of 1 to 9. k is, for example, 9. In addition, it is preferable to use a compound having a small molecular weight as the constitutional unit (b) since the dispersion ratio can be reduced and a compound having a more uniform molecular weight can be obtained. As such a compound from which the constitutional unit (b) is derived, acrylamide is more preferable. 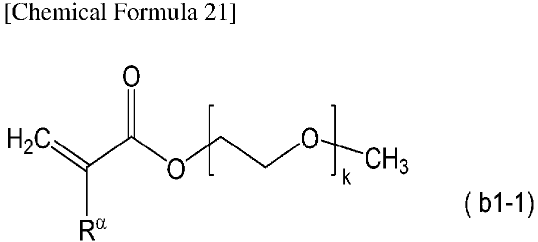 [In the formula, k represents an integer of 1 to 12.]

The constitutional unit (b) may be used alone or in combination of two or more types thereof.

In a case where the copolymer (C) is a compound represented by General Formula (b-2), Lc¹ in General Formula (b-2) represents an (a + b)-valent linking group, Lc¹ is not particularly limited, and examples thereof include a hydrocarbon group which may have an (a + b)-valent substituent. The hydrocarbon group preferably has 1 to 10 carbon atoms, more preferably has 1 to 6 carbon atoms, still more preferably has 5 carbon atoms, even still more preferably has 1 to 3 carbon atoms, and particularly preferably has 1 or 2 carbon atoms. The hydrocarbon group which may have a substituent may be an aromatic hydrocarbon group which may have a substituent or an aliphatic hydrocarbon group which may have a substituent, but is preferably an aliphatic hydrocarbon group which may have a substituent. The hydrocarbon group which may have a substituent may be saturated or unsaturated, but is preferably saturated.

The hydrocarbon group in Lc¹ may or may not have a substituent. Examples of the substituent which may be contained in the hydrocarbon group in Lc¹ include a carboxy group, an amino group, a hydroxyl group, an alkoxy group, and the like.

Specific examples of Lc¹ include a carbon atom.

In General Formula (b-2), a and b are each independently an integer of 1 or more as long as an atomic valence is allowed. a and b are preferably an integer of 1 to 4 and more preferably an integer of 1 to 3. Specific examples thereof include a combination of a = 3 and b = 1.

The copolymer (C) is preferably a copolymer (hereinafter, also referred to as a "copolymer (C01)") having the constitutional unit (a) represented by General Formula (a-1) and the constitutional unit (b) represented by General Formula (b-1), or a compound (hereinafter, also referred to as a "copolymer (C02)") represented by General Formula (b-2).

The molar ratio of the constitutional unit (a) to the constitutional unit (b) (constitutional unit (a): constitutional unit (b)) is preferably 10:1 to 1:20 and more preferably 10:1 to 1:10. In a case where the molar ratio of the constitutional unit (a) to the constitutional unit (b) is within the above-described range, the expression levels of αSMA and collagen (Col1a2) in the myofibroblasts can be further suppressed, and the expression levels of HMOX1, MMP, ANGPTL4, and SOD2 can be further improved.

In a case where the copolymer (C) is the copolymer (C01), the constitutional unit (a): constitutional unit (b) (molar ratio) is preferably 1:20 to 8:2, more preferably 1:10 to 8:2, still more preferably 1:9 to 6:4, even still more preferably 1:9 to 5:5, and particularly preferably 1:9 to 4:6. From the viewpoints of promoting the inactivation of the myofibroblasts or suppressing the activation thereof, the proportion of the constitutional unit (a) is preferably smaller than that of the constitutional unit (b).

Examples of the total number of the constitutional units (a) and the constitutional units (b) in Copolymer (C01) include 10 to 50, and 10 to 40 is preferable and 10 to 35 is more preferable. Examples of the number of the constitutional units (a) in Copolymer (C01) include 1 to 20, and 1 to 15 is preferable, 1 to 13 more preferable, 1 to 12 is still more preferable, and 1 to 10 is particularly preferable. Examples of the number of the constitutional units (b) in Copolymer (C01) include 1 to 30, 3 to 30 is preferable, 4 to 30 is more preferable, 8 to 30 is still more preferable, 10 to 26 is even still more preferable, and 15 to 26 is particularly preferable. From the viewpoints of promoting the inactivation of the myofibroblasts or suppressing the activation thereof, the number of the constitutional units (a) is preferably smaller than the number of the constitutional units (b). In Copolymer (C01), for example, it is preferable that one constitutional unit (a) be introduced with respect to 1 to 10 (for example, 2 to 5) constitutional units (b), it is more preferable that one constitutional unit (a) be introduced with respect to 3 to 10 (for example, 3 to 4) constitutional units (b), and it is still more preferable that one constitutional unit (a) be introduced with respect to 5 to 10 (for example, 5 to 9) constitutional units (b).

In a case where Copolymer (C) is the copolymer (C02), the constitutional unit (a): constitutional unit (b) (molar ratio) is preferably 1:5 to 5:1, more preferably 1:4 to 4:1, and still more preferably 1:3 to 3:1. From the viewpoints of promoting the inactivation of the myofibroblasts or suppressing the activation thereof, the proportion of the constitutional unit (a) is preferably smaller than that of the constitutional unit (b).

Examples of the total number of the constitutional units (a) and the constitutional units (b) in Copolymer (C02) include 2 to 10, and 2 to 6 is preferable and 2 to 4 is more preferable. Examples of the number of the constitutional units (a) in Copolymer (C02) include 1 to 10, and 1 to 6 is preferable, 1 to 4 is more preferable, and 1 to 3 is still more preferable. Examples of the number of the constitutional units (b) in Copolymer (C02) include 1 to 10, and 1 to 6 is preferable, 1 to 4 is more preferable, and 1 to 3 is still more preferable. From the viewpoints of promoting the inactivation of the myofibroblasts or suppressing the activation thereof, the number of the constitutional units (a) is preferably smaller than the number of the constitutional units (b).

Specific examples of Copolymer (C01) are shown below, but Copolymer (C01) is not limited thereto. 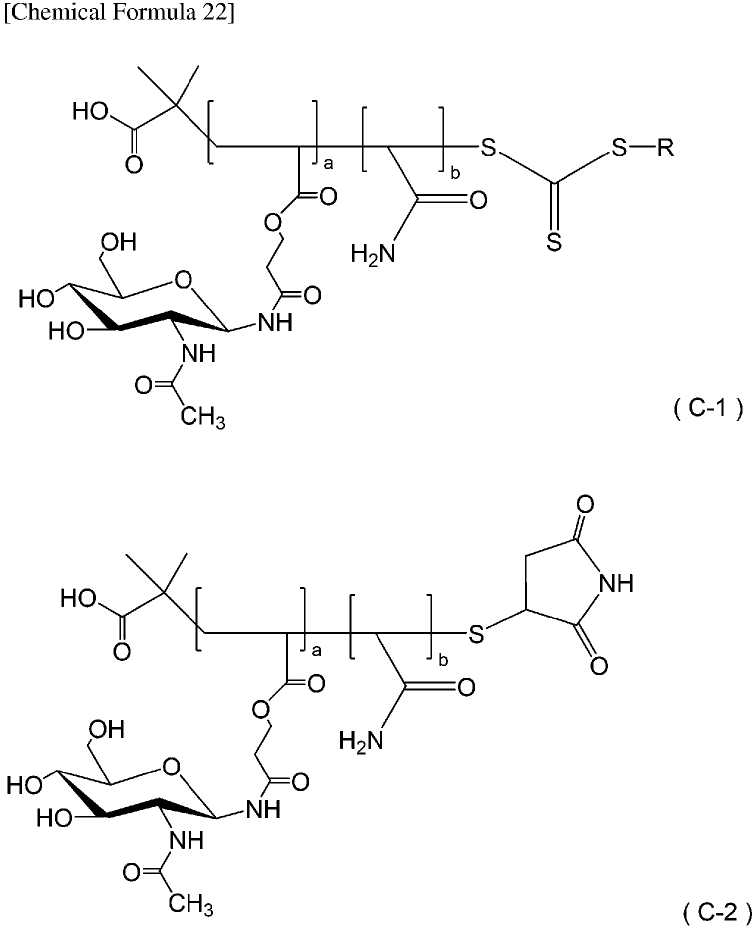 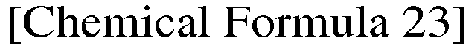 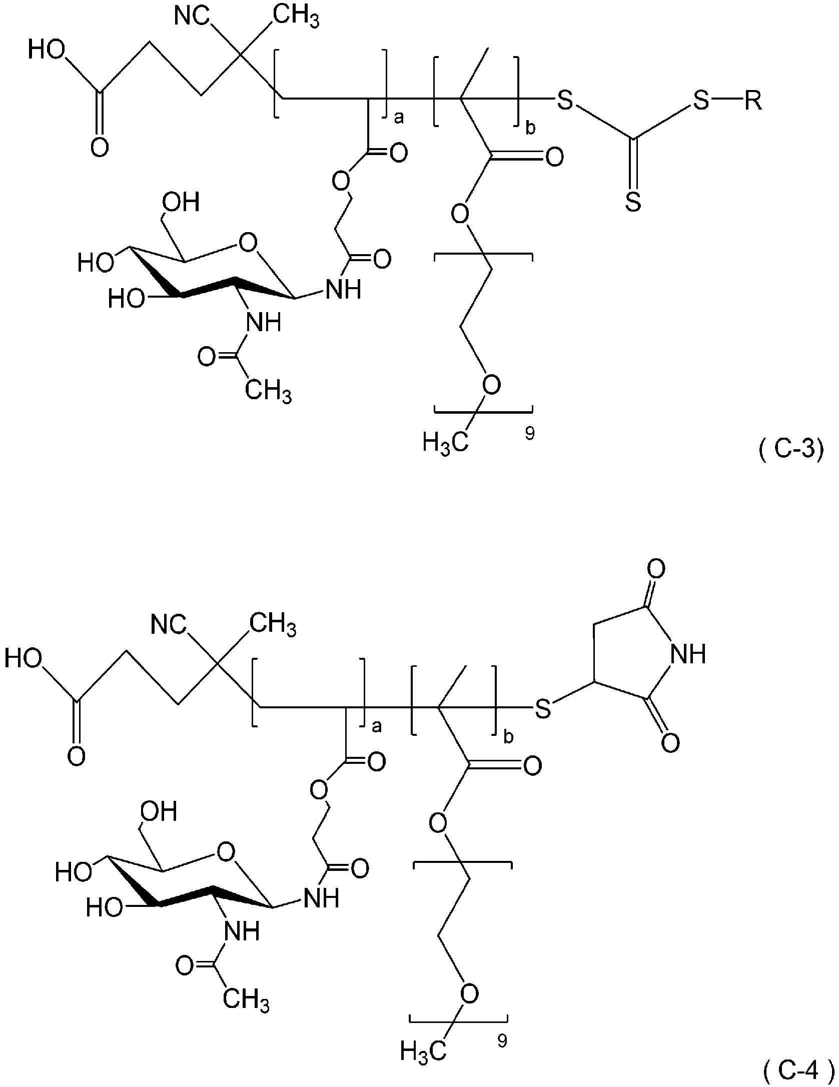 [In the formula, R represents an alkyl group having 1 to 18 carbon atoms, and a and b each independently represents an integer of 1 to 30.]

Specific examples of Copolymer (C02) are shown below, but Copolymer (C02) is not limited thereto. 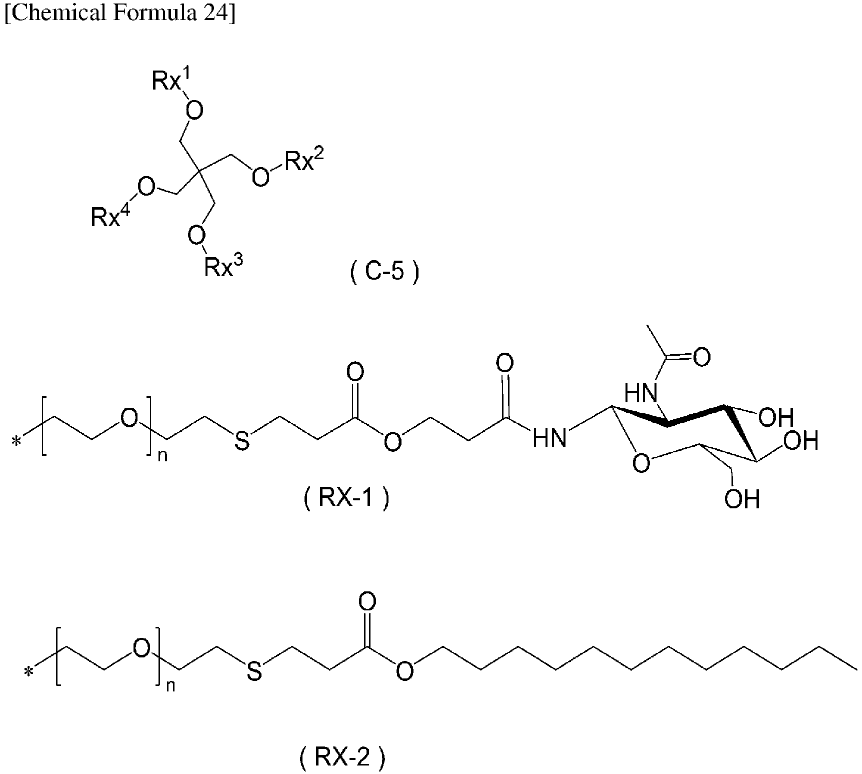 [In the formula, Rx¹ to Rx⁴ each independently represents a group represented by Formula (RX-1) or (RX-2), provided that at least one of Rx¹ to Rx⁴ is a group represented by Formula (RX-1). n represents an integer of 1 or more. * represents a bonding site.]

Examples of n in Formulae (RX-1) and (RX-2) include an integer of 1 to 30, and an integer of 10 to 30 is preferable.

Copolymer (C01) is preferably a random copolymer. The copolymer represented by Formulae (C-1) to (C-4) is a random copolymer.

The weight-average molecular weight (Mw) of the copolymer (C) is not particularly limited and can be appropriately set depending on the constitutional unit (a) and the molecular weight of the constitutional unit. The weight-average molecular weight (Mw) of the copolymer (C) is, for example, preferably in a range of 2,000 to 20,000. In a case where the weight-average molecular weight (Mw) of the copolymer (C) is within the above-described preferred range, the expression levels of αSMA and collagen (Col1a2) in the myofibroblasts can be further suppressed, and the expression levels of HMOX1, MMP, ANGPTL4, and SOD2 can be further improved.

In a case where the copolymer (C) is a copolymer represented by Formula (C-1) or (C-2), the weight-average molecular weight (Mw) is preferably 2,000 to 6,000 and more preferably 3,000 to 5,000. In a case where the copolymer (C) is a copolymer represented by Formula (C-3) or (C-4), the weight-average molecular weight (Mw) is preferably 7,000 to 20,000 and more preferably 7,000 to 16,000. In a case where the copolymer (C) is a copolymer represented by Formula (C-5), the weight-average molecular weight (Mw) is preferably 4,000 to 10,000 and more preferably 5,000 to 8,000.

The number-average molecular weight (Mn) of the copolymer (C) is not particularly limited and can be appropriately set depending on the constitutional unit (a) and the molecular weight of the constitutional unit. The number-average molecular weight (Mn) of the copolymer (C) is, for example, preferably in a range of 2,000 to 15,000. In a case where the number-average molecular weight (Mn) of the copolymer (C) is within the above-described preferred range, the expression levels of αSMA and collagen (Col1a2) in the myofibroblasts can be further suppressed, and the expression levels of HMOX1, MMP, ANGPTL4, and SOD2 can be further improved.

In a case where the copolymer (C) is a copolymer represented by Formula (C-1) or (C-2), the number-average molecular weight (Mn) is preferably 2,000 to 6,000 and more preferably 3,000 to 5,000. In a case where the copolymer (C) is a copolymer represented by Formula (C-3) or (C-4), the number-average molecular weight (Mn) is preferably 4,500 to 15,000 and more preferably 8,000 to 12,000. In a case where the copolymer (C) is a copolymer represented by Formula (C-5), the number-average molecular weight (Mn) is preferably 4,000 to 10,000 and more preferably 5,000 to 8,000.

The ratio (Mw/Mn) of the weight-average molecular weight (Mw) to the number-average molecular weight (Mn) of the copolymer (C) is preferably in a range of 1.0 to 1.7. The Mw/Mn is more preferably 1.0 to 1.6.

### <<Method for manufacturing copolymer (C)>>

The method for manufacturing a copolymer (C) is not particularly limited, and radical polymerization, living radical polymerization, reversible addition-fragmentation chain transfer polymerization method (RAFT polymerization method) using a chain transfer agent (RAFT agent), or the like can be used. For example, the copolymer (C) can be manufactured by mixing a monomer from which the constitutional unit (a) is derived and a monomer from which the constitutional unit (b) is derived, and copolymerizing the monomers by the method as described above.

In the copolymerization reaction, examples of the mixing molar ratio of the monomer from which the constitutional unit (a) is derived to the monomer from which the constitutional unit (b) is derived include a range of 1:20 to 10:1 or 1:10 to 10:1. The mixing molar ratio of the monomer from which the constitutional unit (a) is derived to the monomer from which the constitutional unit (b) is derived is preferably 1:20 to 8:2, more preferably 1:10 to 8:2, still more preferably 1:9 to 6:4, even still more preferably 1:9 to 5:5, and particularly preferably 2:9 to 4:6 or 2:8 to 4:6.

In a case of Copolymer (C01), it is preferable to use the RAFT polymerization method as the polymerization method since the molecular weight distribution of Copolymer (C01) can be narrowly controlled. For example, Copolymer (C01) can be obtained by mixing the monomer from which the constitutional unit (a) is derived and the monomer from which the constitutional unit (b) is derived, and performing RAFT polymerization in a solvent such as DMSO in the presence of a RAFT agent.

After the RAFT polymerization, a terminal group represented by -S-C(=S)-Z (Z is an organic group) derived from the RAFT agent may be reduced with a reducing agent such as sodium borohydride to obtain a thiol group. Furthermore, an unsaturated group-containing compound having reactivity with a thiol group may be reacted with the thiol group to introduce any functional group. For example, a succinimide group may be introduced into the terminal of Copolymer (C01) by reacting maleimide with a thiol group.

Copolymer (C02) can be obtained by reacting a polyfunctional compound having a plurality of functional groups with a compound containing an N-acetylglucosamine group (hereinafter, also referred to as a "GlcNac compound") and a compound not containing an N-acetylglucosamine group (hereinafter, also referred to as a "non-GlcNac compound"). As the GlcNac compound and the non-GlcNac compound, a compound having a functional group reactive with the functional group of the polyfunctional compound may be used. For example, in a case where the polyfunctional compound has a thiol group, as a GlcNac compound and a non-GlcNac compound, a compound having a vinyl group can be used. In a case where the polyfunctional compound has a thiol group, it is preferable to reduce the thiol group before the reaction.

The antifibrotic agent of the present embodiment contains the copolymer (C) of a constitutional unit (a) containing an N-acetylglucosamine group, and another constitutional unit (b), thereby exhibiting an inactivating action and/or an activation suppressing action on myofibroblasts. More specifically, the copolymer (C) suppresses the expression levels of collagen (Col1a2) and αSMA in the myofibroblasts, thereby suppressing tissue fibrogenesis. Furthermore, the copolymer (C) protects cells against oxidative stress and alleviates tissue fibrogenesis by increasing the expression level of HMOX1 in myofibroblasts. The copolymer (C) increases the expression level of MMP1, thereby promoting the decomposition of collagen deposited in the fibrous tissue and suppressing tissue fibrogenesis. The copolymer (C) increases the expression level of ANGPTL4, thereby suppressing tissue fibrogenesis by exhibiting a wound healing action and an anti-inflammatory action. The copolymer (C) increases the expression level of SOD2, thereby suppressing tissue fibrogenesis by an antioxidant action.

The copolymer (C) has the constitutional unit (b) in addition to the constitutional unit (a), thereby the number of the constitutional units (a) contained in one molecule of the copolymer can be reduced. As a result, the N-acetylglucosamine group contained in one molecule of the copolymer can be reduced. In this manner, in the copolymer (C), the action of suppressing the expression of collagen (Col1a2) and αSMA and the action of promoting the expression of MMP1, HMOXA, ANGPTL4, and SOD2 are further improved.

Therefore, the antifibrotic agent of the present embodiment has a better inactivating action and/or a better activation suppressing action of myofibroblasts.

### (Pharmaceutical composition for treating fibrosis)

The pharmaceutical composition for treating fibrosis according to the present embodiment contains the above-described antifibrotic agent as an active ingredient. The "active ingredient" is an ingredient that is effective for treating fibrosis, and more specifically, an ingredient having an action of improving or alleviating fibrosis and/or suppressing the progression of fibrosis. The pharmaceutical composition for treating fibrosis according to the present embodiment contains the copolymer (C) in a therapeutically effective amount as an active ingredient. The therapeutically effective amount refers to the amount of the copolymer (C) that can exhibit the above-described therapeutic effect on the treatment of fibrosis.

The pharmaceutical composition for treating fibrosis according to the present embodiment can be administered orally or parenterally (intravenous administration, subcutaneous administration, transdermal administration, transpulmonary administration, transmucosal administration, rectal administration, or the like) to humans or mammals other than humans (for example, rabbits, cats, dogs, cows, sheep, monkeys, and the like). The pharmaceutical composition for treating fibrosis according to the present embodiment can be prepared by mixing the copolymer (C) with a pharmaceutically acceptable carrier (excipients, binders, disintegrators, disintegration aids, lubricants, wetting agents, and the like), and additives that are generally used for oral administration or parenteral administration, and formulating the mixture into a desired form. Examples of the dosage form of the pharmaceutical composition for treating fibrosis according to the present embodiment include a granule preparation, a powder preparation, a tablet preparation (including a sugar-coated tablet preparation), a pill, a buccal preparation, a capsule preparation, a syrup preparation, a liquid preparation, an emulsion preparation, a suspension preparation, a cream preparation, an ointment preparation, an eye drop preparation, an injection preparation, a drip preparation, a nasal preparation, a patch preparation, a suppository, and the like.

The content of the copolymer (C) in the pharmaceutical composition for treating fibrosis according to the present embodiment is not particularly limited as long as the expression of αSMA and collagen is suppressed and the effect of improving fibrogenesis is obtained. The content of the copolymer (C) is preferably 0.1% by mass or more, more preferably 1% by mass or more, and still more preferably 10% by mass or more.

### <Fibrosis>

Fibrosis described in the present specification means stiffening accompanying tissue dysfunction due to excessive migration and proliferation of fibroblasts generated in the process of compensating for tissue parenchymal cell shedding or tissue function deterioration that occurs by a stress such as chemical stimulation of a drug and the like, an excessive pressure load, an inflammatory reaction, and the like; and the subsequent synthetic deposition of an extracellular matrix protein. The type or the onset site of the inducing stimulus is not particularly limited. Examples of such tissue fibrogenesis disease include fibrosis of visceral tissues such as the lung, the bladder, the kidney, the heart, and the liver.

Examples of fibrosis targeted by the pharmaceutical composition for treating fibrosis of the present embodiment include tissue fibrotic diseases caused by administration of an agent such as an antitumor agent, an antibiotic, an antibacterial agent, an antiarrhythmic agent, an anti-inflammatory agent, an antirheumatic agent, an interferon, and Sho-saiko-to; and tissue fibrogenesis diseases associated with diseases such as chronic nephritis, interstitial myocarditis, and interstitial cystitis. Specific examples of such fibrosis include pulmonary fibrosis caused by side effects of bleomycin administration, or pulmonary fibrosis occurring during or after interstitial pneumonia; bladder fibrosis or bladder cirrhosis occurring during interstitial cystitis; renal fibrosis caused by genetic abnormalities and the like, and renal failure (renal sclerosis); endocardial fibrosis caused by remodeling after myocardial infarction; liver fibrosis caused by damage to hepatic cells; non-alcoholic steatohepatitis (NASH), and concomitant portal hypertension and cirrhosis; keloids caused by excessive tissue repair; and sclerosing peritonitis, prostatic hypertrophy, sclerosis, uterine smooth myoma, retroperitoneal fibrosis, and myeloid fibrosis.

The pharmaceutical composition for treating fibrosis according to the present embodiment may be a pharmaceutical composition which suppresses phosphorylation of STAT3 to decrease an expression level of αSMA or collagen, increases an expression level of MMP1, HMOX1, and ANGPTL4, and restores myofibroblasts or activated astrocytes to normal fibroblasts or normal astrocytes to improve fibrogenesis. In the case of fibrosis consisting of myofibroblasts and activated astrocytes, for example, in the case of the liver, the activated astrocytes are transformed into myofibroblast-like cells, and the extracellular matrix is produced causing fibrogenesis to progress. However, by using the pharmaceutical composition for treating fibrosis according to the present embodiment, it is possible to improve liver fibrogenesis and to improve liver function and to suppress the occurrence of liver cancer. Similarly, it is also possible to use the fibrosis therapeutic drug for pancreatic fibrogenesis and the like.

### (Method for inactivating myofibroblasts)

The method for inactivating myofibroblasts of the present embodiment includes binding the above-described antifibrotic agent to the myofibroblasts. In the method of the present embodiment, a copolymer (C) can be used as the antifibrotic agent.

The "inactivation of myofibroblasts" refers to the occurrence of at least one phenomenon selected from the group consisting of, in the myofibroblasts, a decrease in a collagen expression level, a decrease in an αSMA expression level, an increase in an MMP1 expression level, an increase in an HMOX1 expression level, and an increase in an ANGPTL4 expression level. In the inactivation of the myofibroblasts, it is preferable that two or more of the phenomena occur, it is more preferable that three or more of the phenomena occur, it is still more preferable that four or more of the phenomena occur, and it is particularly preferable that all the phenomena occur.

The binding of the antifibrotic agent to the myofibroblasts can be performed by bringing the antifibrotic agent into contact with the myofibroblasts. The contact of the antifibrotic agent with the myofibroblasts may be performed in vitro or may be performed in vivo. The in vitro contact can be performed by adding an antifibrotic agent to a culture solution of the myofibroblasts. The in vivo contact can be performed by administering an antifibrotic agent to a living body.

In a case of being in vitro, examples of the amount of the antifibrotic agent to be added to the myofibroblasts include 10 to 500 µg/mL, 50 to 300 µg/mL is preferable, and 50 to 100 µg/mL is more preferable with respect to the culture solution of the myofibroblasts.

In a case of being in vivo, the dose to be administered to the living body can be appropriately set according to the biological species, body weight, sex, age, and the like of the living body. Examples of the dose of the antifibrotic agent to be administered to a living body include 10 to 1,000 µg/kg.

In a case of being in vitro, the culture of the myofibroblasts can be performed in an environment of about 37°C (37 ± 2°C) and a carbon dioxide concentration of about 5% by volume (5 ± 2% by volume).

### (Other Embodiments)

In one embodiment, the present invention provides a treatment method for fibrosis, including administering an effective amount of the above-described antifibrotic agent to a patient or a diseased animal in need of treatment. As the antifibrotic agent, a copolymer (C) can be used. Examples of the fibrosis to which the agent is applied include the same ones as those described in the above-described "Pharmaceutical composition for treating fibrosis".

In one embodiment, the present invention provides the use of a copolymer (C) for manufacturing an antifibrotic agent.

In one embodiment, the present invention provides the use of a copolymer (C) for manufacturing a pharmaceutical composition for treating fibrosis.

In one embodiment, the present invention provides a copolymer (C) for use in the treatment of fibrosis.

In one embodiment, the present invention provides the use of a copolymer (C) for treating fibrosis.

### Example

Hereinafter, the present invention will be described with reference to Examples, but the present invention is not limited to such Examples.

### (Preparation of myofibroblast)

Primary cultured human dermal fibroblasts (manufactured by Takara Bio Inc.) separated from the skin of an adult confirmed to have no mycoplasma contamination were cultured in an HFDM-1 (+) culture medium (manufactured by Cell Science & Technology Institute, Inc.) containing 2 mM L-glutamine (manufactured by FUJIFILM Wako Pure Chemical Corporation) in a 37°C, 5 v/v% CO₂ humidified incubator. Subsequently, 10 ng/mL TGF-β was added to the culture medium to obtain myofibroblasts. In order to obtain these myofibroblasts, human dermal fibroblasts maintained in 3 to 6 subcultures were used.

### (Confirmation of presence or absence of mycoplasma contamination)

The human dermal fibroblasts to be used were confirmed to be free of contamination with mycoplasma using Mycoplasma Detection Kit for Endpoint PCR, OneStep, VendorGeM (manufactured by Minerva Biolabs GmbH).

### (Synthesis of AC-GlcNAc monomer)

5 g of GlcNac was dissolved in 50 mL of water, and NH₄CO₃ was added thereto until saturation. The mixture was stirred in an eggplant flask and incubated in an open system with a lid open at 30°C to 35°C for 4 to 5 days, and then NH₄CO₃ was appropriately added thereto when the precipitation of NH₄CO₃ had disappeared. The synthesis of GlcNac-NH2 was confirmed by thin layer chromatography (TLC). Water was added to the reaction solution 4 to 5 days later, and an evaporator (30°C) was used to remove excess NH₄CO₃. This operation was repeatedly performed many times until the odor disappeared. After using the evaporator, freeze-drying was performed. Next, GlcNAc-NH₂ (4.5 mol: about 1 g) was dissolved in dimethyl sulfoxide (DMSO) (10 mL), and 2-carboxyethyl acrylate (4.5 mmol) was added thereto. After the dissolution, 4-(4,6-dimethoxy-1,3,5-triazin-2-yl)-4-methylmorpholinium chloride (DMT-MM) (6.8 mmol) was added thereto, and the mixture was incubated at room temperature for 18 hours. After the incubation, the reactant was added dropwise to 200 to 300 mL of chloroform. The obtained precipitate was collected with a Kiriyama funnel. The collected precipitate was dissolved in methanol, the insoluble matter was removed with a Kiriyama funnel, and the fraction dissolved in methanol was collected. An evaporator was used to remove methanol. The solid matter from which methanol was removed was dissolved in water. Thereafter, the purification was performed by preparative high-performance liquid chromatography (HPLC) (water/acetonitrile) to obtain an AC-GlcNAc monomer (molecular weight of 346) represented by the following chemical formula (1).

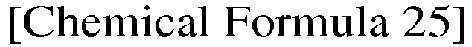

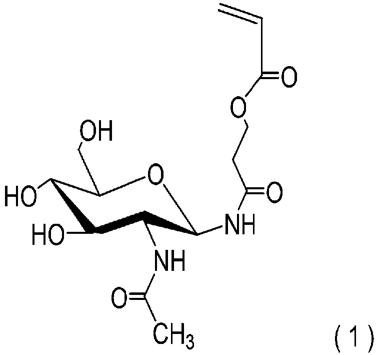

### (Manufacture of Copolymer 1)

40 mg (0.116 mmol) of an AC-GlcNAc monomer and 2.1 mg (0.029 mmol) of acrylamide (molecular weight of 71) were dissolved in 250 µL of DMSO. 5.3 mg (0.0145 mmol) of a RAFT agent (2-(dodecylthiocarbonothioylthio)-2-methylpropanoic acid) and 1 mg of azobisisobutyronitrile (AIBN) were mixed into the solution. This solution was repeatedly subjected to freeze-thawing about 5 times while degassing to remove dissolved oxygen. Thereafter, the mixture was sealed with argon gas and heated for about 17 hours at 65°C while stirring to perform polymerization. After the polymerization, this solution was suspended in isopropanol to collect the polymerized substance as a precipitate. The precipitate was dissolved in water and dialyzed with water for 17 hours using a dialysis membrane (MWCO of 1,000). After dialysis, the solution was freeze-dried to obtain Copolymer 1 (Formula (C1-1)).

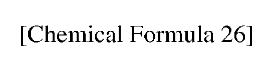

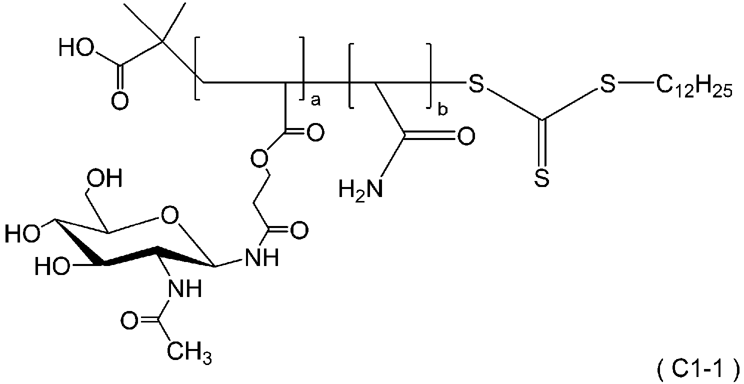

Copolymer 1 had a weight-average molecular weight (Mw) of 5,000, a number-average molecular weight (Mn) of 4,400, Mw/Mn of 1.14, and a molar ratio of an AC-GlcNAc unit to an acrylamide unit of 13:3. Each of these average molecular weights was measured under the following conditions using a Gel Permeation Chromatography (GPC) device (product name: LC-9110G NEXT, manufactured by Japan Analytical Industry Co., Ltd.). The column was JAIGEL-GS510 and the eluate was 200 mM sodium nitrate/acetonitrile = 80/20. The flow rate was 1 mL/min, the detector was an RI detector, and the column temperature was 40°C. A standard curve of the molecular weight was obtained with a flurane. Copolymer 1 had thirteen AC-GlcNAc units and three acrylamide units.

### (Manufacture of Copolymer 2)

Copolymer 2 was obtained by the same method as in (Manufacture of Copolymer 1), except that the amount of the AC-GlcNac monomer (molecular weight of 346) used was 30 mg (0.087 mmol) and the amount of the acrylamide (molecular weight of 71) used was 4.2 mg (0.059 mmol). Copolymer 2 had a weight-average molecular weight (Mw) of 4,900, a number-average molecular weight (Mn) of 4,500, Mw/Mn of 1.09, and a molar ratio of an AC-GlcNAc unit to an acrylamide unit of 12:8.

### (Manufacture of Copolymer 3)

40 mg (0.116 mmol) of an AC-GlcNAc monomer (molecular weight of 346) and 14.4 mg (0.029 mmol) of poly(ethylene glycol) methyl ether methacrylate (molecular weight of 500) were dissolved in 250 µL of DMSO. 5.3 mg (0.0145 mmol) of a RAFT agent (4-cyano-4-[(dodecylsulfanylthiocarbonyl)sulfanyl]pentanoic acid) and 1 mg of AIBN were mixed with the solution. This solution was repeatedly subjected to freeze-thawing about 5 times while degassing to remove dissolved oxygen. Thereafter, the mixture was sealed with argon gas and heated for about 17 hours at 65°C while stirring to perform polymerization. After the polymerization, this solution was suspended in isopropanol to collect the polymerized substance as a precipitate. The precipitate was dissolved in water and dialyzed with water for 17 hours using a dialysis membrane (MWCO of 1,000). After dialysis, the solution was freeze-dried to obtain Copolymer 3 (Formula (C1-3)).

Copolymer 3 had a weight-average molecular weight (Mw) of 7,100, a number-average molecular weight (Mn) of 4,700, Mw/Mn of 1.51, and a molar ratio of an AC-GlcNAc unit to a poly(ethylene glycol) methyl ether methacrylate unit of 15:4. Each of these average molecular weights was measured under the following conditions using a Gel Permeation Chromatography (GPC) device (product name: LC-9110G NEXT, manufactured by Japan Analytical Industry Co., Ltd.). The column was JAIGEL-GS510 and the eluate was 200 mM sodium nitrate/acetonitrile = 80/20. The flow rate was 1 mL/min, the detector was an RI detector, and the column temperature was 40°C. A standard curve of the molecular weight was obtained with a flurane.

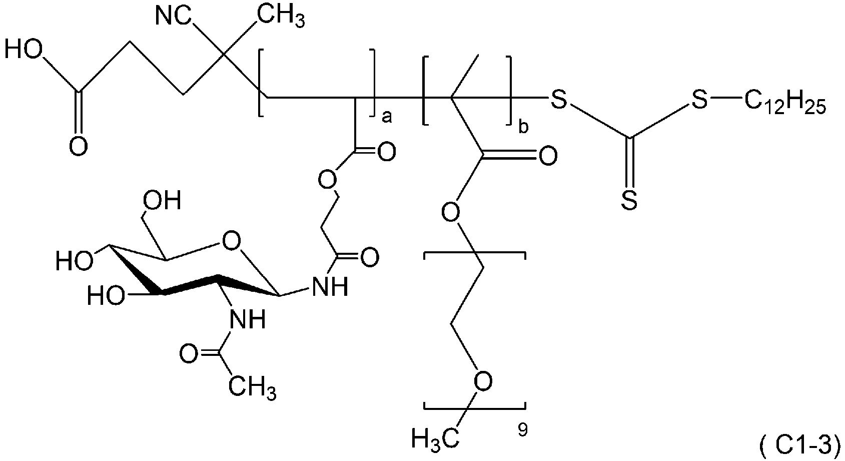

### (Manufacture of Copolymer 4)

Copolymer 4 was obtained by the same method as in (Manufacture of copolymer 3), except that the amount of the AC-GlcNac monomer (molecular weight of 346) used was 30 mg (0.087 mmol) and the amount of the poly(ethylene glycol) methyl ether methacrylate (molecular weight of 500) used was 28.8 mg (0.0576 mmol). Copolymer 4 had a weight-average molecular weight (Mw) of 10,300, a number-average molecular weight (Mn) of 7,400, Mw/Mn of 1.39, and a molar ratio of an AC-GlcNAc unit to a poly(ethylene glycol) methyl ether methacrylate unit of 15:10.

### (Manufacture of Copolymer 5)

Copolymer 5 was obtained by the same method as in (Manufacture of copolymer 3), except that the amount of the AC-GlcNac10 monomer (molecular weight of 346) used was 20 mg (0.059 mmol) and the amount of the poly(ethylene glycol) methyl ether methacrylate (molecular weight of 500) used was 43.5 mg (0.087 mmol). Copolymer 5 had a weight-average molecular weight (Mw) of 13,000, a number-average molecular weight (Mn) of 9,200, Mw/Mn of 1.41, and a molar ratio of an AC-GlcNAc unit to a poly(ethylene glycol) methyl ether methacrylate unit of 12:18.

### (Manufacture of Copolymer 6)

Copolymer 6 was obtained by the same method as in (Manufacture of copolymer 3), except that the amount of the AC-GlcNac monomer (molecular weight of 346) used was 10 mg (0.029 mmol) and the amount of the poly(ethylene glycol) methyl ether methacrylate (molecular weight of 500) used was 58 mg (0.116 mmol). Copolymer 6 had a weight-average molecular weight (Mw) of 15,100, a number-average molecular weight (Mn) of 11,800, Mw/Mn of 1.28, and a molar ratio of an AC-GlcNAc unit to a poly(ethylene glycol) methyl ether methacrylate unit of 6:26.

### (Manufacture of Polymer 1)

(1) 50 mg of an AC-GlcNAc monomer (molecular weight of 346) was weighed into a 1 mL microtube, and dissolved in 250 µL of DMSO.
(2) About 5.26 mg (1/10 molar equivalents of a monomer) of a RAFT agent (2-(dodecylthiocarbonothioylthio)-2-methylpropanoic acid (DTMPA: manufactured by Sigma) was weighed into a 1 mL microtube, and dissolved in 200 µL of DMSO.
(3) 1 mg (2% with respect to the monomer) of azobisisobutyronitrile (AIBN) was weighed into a microtube and dissolved in 50 µL of DMSO.

200 µL of solution (2) was mixed with 250 µL of solution (1), then 50 µL of solution of (3) was mixed therewith, and the mixture was degassed (the freeze-and-thaw degassing was repeated about three times). Thereafter, the mixture was incubated at 65°C for about 18 hours to polymerize the AC-GlcNAc monomer. After the polymerization, the polymerized substance was precipitated with isopropanol, and after centrifugation, the polymerized substance was collected. The collected substance was dissolved in water, and dialysis (MW 100 to 500) was performed for about 1 day to remove unreacted monomers. After dialysis, freeze-drying was performed to obtain Polymer 1 (Formula (C2-1)).

Polymer 1 had a weight-average molecular weight (Mw) of 4,000, a number-average molecular weight (Mn) of 3,100, and Mw/Mn of 1.29, and these average molecular weights are measured by the same method as that of Copolymer 1.

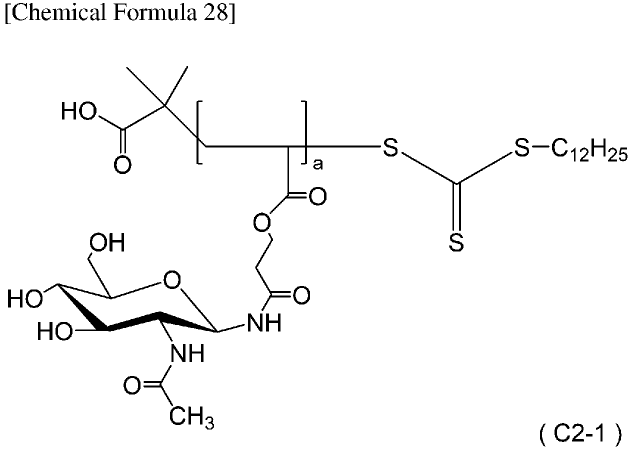

Table 1 summarizes the results of GPC measurement of Copolymer 1 to Copolymer 6 and Polymer 1. In Table 1, in "Mixing molar ratio of monomers" and "Molar ratio of constitutional units", "a" represents a molar ratio of an AC-GlcNAc monomer of an AC-GlcNAc unit, and "b" represents a molar ratio of acrylamide or poly(ethylene glycol) methyl ether methacrylate, or a constitutional unit derived from these.

**[Table 1]**

| | Mixing molar ratio of monomers (a:b) | Mw | Mn | Mw/Mn | Valence | Molar ratio of constitutional units (a:b) |
|---|---|---|---|---|---|---|
| Copolymer 1 | 8:2 | 5,000 | 4,400 | 1.14 | 16 | 13:3 |
| Copolymer 2 | 6:4 | 4,900 | 4,500 | 1.09 | 20 | 12:8 |
| Copolymer 3 | 8:2 | 7,100 | 4,700 | 1.51 | 19 | 15:4 |
| Copolymer 4 | 6:4 | 10,300 | 7,400 | 1.39 | 25 | 15:10 |
| Copolymer 5 | 4:6 | 13,000 | 9,200 | 1.41 | 30 | 12:18 |
| Copolymer 6 | 2:8 | 15,100 | 11,800 | 1.28 | 32 | 6:26 |
| Polymer 1 | - | 4,000 | 3,100 | 1.29 | - | - |

### [Example 1]

The test compound (Copolymer 1, Copolymer 2, or Polymer 1) at each concentration (10 µg/mL, 50 µg/mL, or 100 µg/mL) was added to the myofibroblasts prepared in (Preparation of fibroblast) and the myofibroblasts were cultured in a 37°C, 5% CO₂ humidified incubator for 48 hours.

After the culture, cells were collected. The expression levels of collagen (Col1a2), α-smooth muscle actin (αSMA), heme oxygenase 1 (HMOX1), and β-actin in the collected myofibroblasts were observed by Western blotting. As a control, human dermal fibroblasts cultured without any addition (Control) and myofibroblasts cultured without adding the test compound (10 ng/mL TGF β1 (myofibroblast)) were used.

The results are shown in FIG. 1. The numerical value below each band in FIG. 1 indicates the relative intensity of each band in a case where the signal intensity of the band in Control was set to 100. The signal intensity of each band was standardized by the signal intensity of the band of β-actin. From the results shown in FIG. 1, it was confirmed that the expression levels of collagen (Col1a2) and αSMA were very high in the myofibroblasts cultured without adding the test compound. In the myofibroblasts to which any one of Copolymer 1 or Copolymer 2 was added, it was confirmed that the expression level of collagen (Col1a2) and αSMA was decreased and the expression level of HMOX1 was increased, compared with the myofibroblasts to which Polymer 1 was added, in the comparison in the same addition amount. In particular, in the fibroblasts to which Copolymer 2 was added, it was confirmed that the expression levels of collagen (Col1a2) and αSMA were significantly decreased, and the expression level of HMOX1 was significantly increased.

HMOX1 is a protein that exerts a protective action against various oxidative stresses, and in a case where the expression of this gene is increased, fibrogenesis is relieved by the antioxidant action. Therefore, it is considered that the activation of the myofibroblasts is further suppressed by the decrease in the expression levels of αSMA and collagen and the increase in the expression level of HMOX1.

### [Example 2]

100 µg/mL of the test compound (Copolymer 3, Copolymer 4, Copolymer 5, Copolymer 6, or Polymer 1) was added to the myofibroblasts prepared in (Preparation of myofibroblast) and the myofibroblasts were cultured in a 37°C, 5% CO₂ humidified incubator for 48 hours. After the culture, cells were collected. The expression levels of αSMA, collagen (col1a2), matrix metalloproteinase 1 (MMP1), HMOX1, angiopoietin-like 4 (ANGPTL4), and β-actin in the collected myofibroblasts were observed by Western blotting. As a control, human dermal fibroblasts cultured without any addition (Control) and myofibroblasts cultured without adding the test compound (10 ng/mL TGF β1 (myofibroblast)) were used.

The results are shown in FIG. 2. The numerical value below each band in FIG. 2 indicates the relative intensity of each band in a case where the signal intensity of the band in Control was set to 100. The signal intensity of each band was standardized by the signal intensity of the band of β-actin. From the results shown in FIG. 2, in the myofibroblasts cultured without adding the test compound, it was confirmed that the expression levels of collagen (Col1a2) and αSMA were extremely high and the expression levels of MMP1, HMOX1, and ANGPTL4 could not be confirmed or were extremely low. In the myofibroblasts to which any one of Copolymer 3 to Copolymer 6 was added, it was confirmed that the expression levels of αSMA and collagen (Col1a2) were decreased and the expression levels of MMP1, HMOX1, and ANGPTL4 were increased, compared with the myofibroblasts to which Polymer 1 was added.

MMP1 is an enzyme that decomposes collagen deposited in fibrous tissue. HMOX1 is a protein that exerts a protective action against various oxidative stresses. ANGPTL4 is a protein that promotes wound healing. The addition of any of Copolymer 3 to Copolymer 5 suppresses the expression levels of collagen and α-SMA in the myofibroblasts, thereby suppressing fibrogenesis. Furthermore, the collagen that has been attached is decomposed by an increase in the expression levels of MMP1 and ANGPTL4. Furthermore, the increase in expression levels of HMOX1 and ANGPTL4 promotes wound healing, and the anti-inflammatory action and the antioxidant action relieve fibrogenesis.

In the comparison of Copolymer 3 to Copolymer 6, the smaller the proportion of the AC-GlcNAc unit, the larger the decrease in the expression levels of αSMA and collagen (Col1a2), and the larger the increase in the expression levels of MMP1, HMOX1, and ANGPTL4.

### (Manufacture of Copolymer 7)

Copolymer 7 was obtained by the same method as in (Manufacture of Copolymer 1), except that the amount of the AC-GlcNac monomer (molecular weight of 346) used was 10 mg (0.029 mmol) and the amount of the acrylamide (molecular weight of 71) used was 18.9 mg (0.261 mmol). Copolymer 7 had a weight-average molecular weight (Mw) of 3,800, a number-average molecular weight (Mn) of 3,300, Mw/Mn of 1.15, and a molar ratio of an AC-GlcNAc unit to an acrylamide unit of 4:35.

### (Manufacture of Copolymer 8)

### <Reduction of 4-arm PEG-SH (weight-average molecular weight of 5,000)>

20 mg of 4-arm PEG-SH (weight-average molecular weight of 5,000) (SUNBRIGHT (registered trademark) PTE-050SH; YUKA SANGYO CO., LTD) was dissolved in 500 µL of DMSO. 1 M dithiotreitol (DTT) was added thereto, and the mixture was reacted overnight to reduce SH. Thereafter, 10 mL of ether was added thereto, and centrifugation was performed to remove the ether. This was repeated three times, and the reduced 4-arm PEG-SH was precipitated, and DTT and DMSO were removed.

### <Manufacture of 4-arm-PEG-AC-GlcNAc:C12 (3:1)>

The reduced 4-arm PEG-SH (20 mg) was dissolved in 500 µL of DMSO (degasified and replaced with argon). 8.3 mg (2 molar equivalent to three molecules of the SH group of 4-arm PEG-SH) of an AC-GlcNac monomer and 1.92 mg (2.2 µL) (2 molar equivalent to one molecule of the SH group of 4-arm PEG-SH) of dodecyl acrylate were added thereto, 10 µL of triethylamine was further added thereto, and the mixture was reacted at 65°C overnight with stirring. After the reaction overnight, the reactant was precipitated with ether, and the unreacted material was removed by centrifugation to collect the precipitate. Thereafter, dialysis was performed overnight with a dialysis membrane having a molecular weight of 1,000, and freeze drying was performed to obtain Copolymer 8 (Formula (C1-8)).

The weight-average molecular weight (Mw) of Copolymer 8 was 6,300, and the molar ratio of the AC-GlcNAc unit, the 4-arm PEG-SH unit, and the dodecyl acrylate unit was 3:1:1. In the following formulae, n is about 10 to 30. 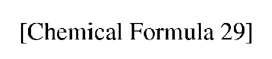 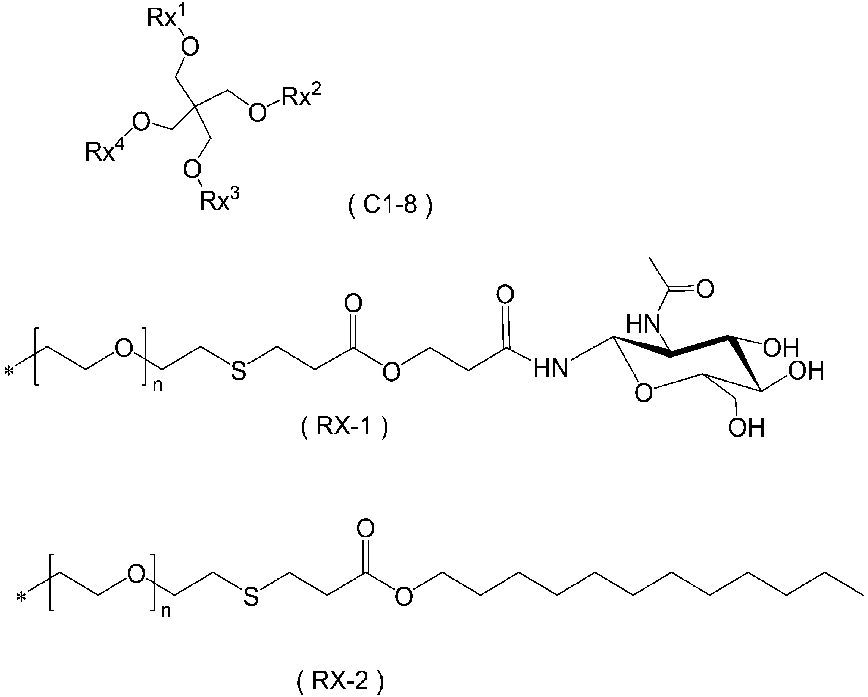 [In the formula, Rx1 to Rx3 each represents a group represented by Formula (RX-1), and Rx4 represents a group represented by Formula (RX-2). n represents an integer of 1 or more.]

The measurement results of Copolymer 7 and Copolymer 8 by GPC are summarized in Table 2. In Table 2, in "Mixing molar ratio of monomers" and "Molar ratio of constitutional units", "a" represents a molar ratio of AC-GlcNAc monomer or AC-GlcNAc unit, "b" represents a molar ratio of 4-arm PEG-SH or a 4-arm PEG-SH unit, and "c" represents a molar ratio of dodecyl acrylate unit.

**[Table 2]**

| | Mixing molar ratio of monomers (a:b) or (a:b:c) | Mw | Mn | Mw/Mn | Valence | Molar ratio of constitutional units (a:b) or (a:b:c) |
|---|---|---|---|---|---|---|
| Copolymer 7 | 1:9 | 3,800 | 3,300 | 1.15 | 39 | 4:35 |
| Copolymer 8 | 3:1:1 | 6,300 | 6,300 | 1 | 3 | 3:1:1 |

### [Example 3]

The culture of the myofibroblasts was performed by the same method as in Example 2, except that a 100 µg/mL test compound was changed to a 10 µg/mL test compound (copolymer 7). After the culture, cells were collected. The expression levels of αSMA, collagen (Colla2), matrix metalloproteinase 1 (MMP1), angiopoietin-like 4 (ANGPTL4), HMOX1, superoxide dismutase 2 (SOD2), and β-actin in the collected myofibroblasts were observed by Western blotting. As a control, human dermal fibroblasts cultured without any addition (Control) and myofibroblasts cultured without adding the test compound (10 ng/mL TGF (myofibroblast)) were used.

The results are shown in FIG. 4. The numerical value below each band in FIG. 4 indicates the relative intensity of each band in a case where the signal intensity of the band in Control was set to 100. From the results shown in FIG. 4, in the myofibroblasts cultured without adding the test compound, it was confirmed that the expression levels of collagen (Col1a2) and αSMA were extremely high and the expression levels of MMP1, HMOX1, ANGPTL4, and SOD2 could not be confirmed or were extremely low. In the myofibroblasts to which Copolymer 7 was added, it was confirmed that the expression levels of αSMA and collagen (Col1a2) were decreased, and the expression levels of MMP1, HMOX1, ANGPTL4, and SOD2 were increased.

SOD2 is an enzyme that removes active oxygen. In Copolymer 7, at a concentration lower than that of each copolymer added in Example 2, the expression levels of collagen and αSMA were suppressed and the expression levels of MMP1, ANGPTL4, and SOD2 were increased in the myofibroblasts. The expression levels of collagen and α SMA were suppressed, thereby fibrogenesis was suppressed. Furthermore, the collagen that had been attached was decomposed by an increase in the expression levels of MMP1 and ANGPTL4. Furthermore, the increase in expression levels of HMOX1, ANGPTL4, and SOD2 promoted wound healing, and the anti-inflammatory action and the antioxidant action relieved fibrogenesis.

### [Example 4]

100 µg/mL of the test compound (Copolymer 8) was added to the myofibroblasts prepared in (Preparation of myofibroblast) and the myofibroblasts were cultured in a 37°C, 5% CO₂ humidified incubator for 48 hours. After the culture, cells were collected. The expression levels of αSMA, collagen (Col1a2), and β-actin in the collected myofibroblasts were observed by Western blotting. As a control, human dermal fibroblasts cultured without any addition (Control) and myofibroblasts cultured without adding the test compound (10 ng/mL TGF (myofibroblast)) were used.

The results are shown in FIG. 5. The numerical value below each band in FIG. 5 indicates the relative intensity of each band in a case where the signal intensity of the band in Control was set to 100. The signal intensity of each band was standardized by the signal intensity of the band of β-actin. From the results shown in FIG. 5, it was confirmed that the expression levels of collagen (Col1a2) and αSMA were very high in the myofibroblasts cultured without adding the test compound. In the myofibroblasts to which Copolymer 8 was added, it was confirmed that the expression levels of αSMA and collagen (Col1a2) were decreased.

### INDUSTRIAL APPLICABILITY

The antifibrotic agent of the embodiment can suppress the expression levels of αSMA and collagen of myofibroblasts and activated astrocytes, and can be used for tissue repair.

Hereinabove, preferable examples of the present invention have been described above, but the present invention is not limited to these examples. Configurations can be added, omitted, and replaced, and other modifications can be made within a range not departing from the gist of the present invention. The present invention is not limited by the above description, but only by the scope of the appended claims.

## Claims

1. An antifibrotic agent comprising:
a copolymer having a constitutional unit (a) containing an N-acetylglucosamine group and a constitutional unit (b) containing a structure represented by General Formula (b) (provided that a constitutional unit corresponding to the constitutional unit (a) is excluded),
[Chemical Formula 1]
*-Yb-Rb (b)
[in the formula, Yb represents a divalent linking group including an oxygen atom, Rb represents a hydrogen atom or an organic group, and * represents a bonding site].

2. The antifibrotic agent according to Claim 1,
wherein the constitutional unit (b) is a constitutional unit represented by General Formula (b-1), 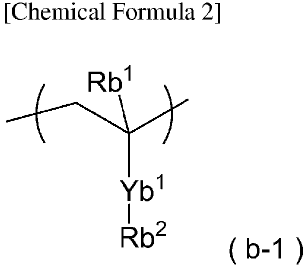 [in the formula, Rb¹ represents a hydrogen atom or an organic group, Yb¹ represents -C(=O)-O- or -C(=O)-NH-, and Rb² represents a hydrogen atom or an organic group].

3. The antifibrotic agent according to Claim 1,
wherein the copolymer is a compound represented by General Formula (b-2), 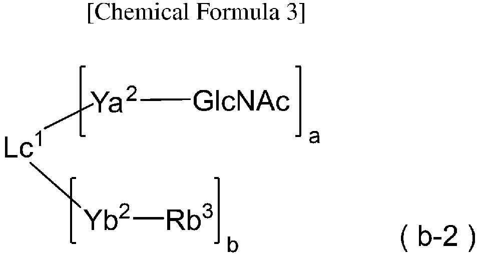 [in the formula, Lc¹ represents an (a + b)-valent linking group, Ya² and Yb² each independently represents a divalent linking group including an oxygen atom, GlcNAC represents an N-acetylglucosamine group, Rb³ represents an organic group, a and b each independently represents an integer of 1 or more as long as an atomic valence is allowed, in a case where a is an integer of 2 or more, two or more Ya²'s may be the same as or different from each other, and in a case where b is an integer of 2 or more, 2 or more Yb²'s may be the same as or different from each other and 2 or more Rb³'s may be the same as or different from each other].

4. The antifibrotic agent according to any one of Claims 1 to 3,
wherein a molar ratio of the constitutional unit (a) to the constitutional unit (b) is 10:1 to 1:20.

5. The antifibrotic agent according to Claim 2,
wherein Rb² in General Formula (b-1) includes at least one structure selected from the group consisting of an ether bond, an ester bond, a urethane bond, and an amide bond.

6. The antifibrotic agent according to any one of Claims 1 to 3,
wherein the constitutional unit (a) is a constitutional unit containing a structure represented by Formula (a1-1-1), 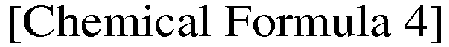 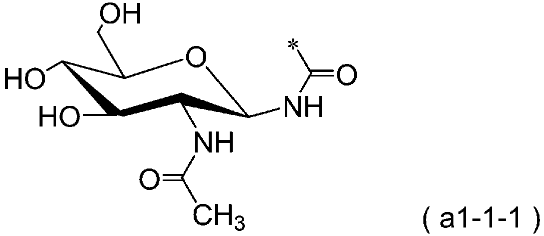 [in the formula, * represents a bonding site].

7. The antifibrotic agent according to Claim 2,
wherein the constitutional unit (a) is a constitutional unit represented by Formula (a-1-1),

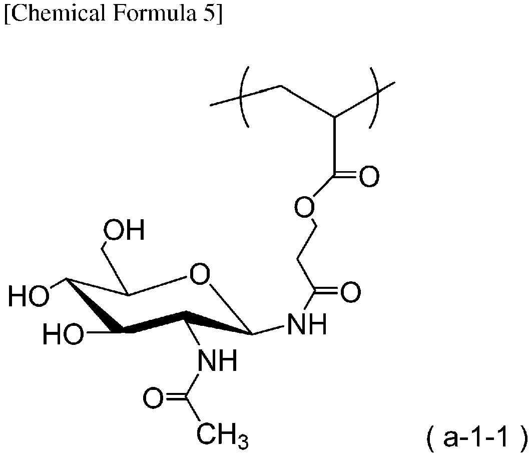

8. The antifibrotic agent according to Claim 2,
wherein the constitutional unit (b) is a constitutional unit derived from acrylamide or a compound represented by General Formula (b1-1), 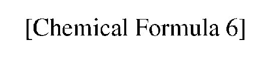 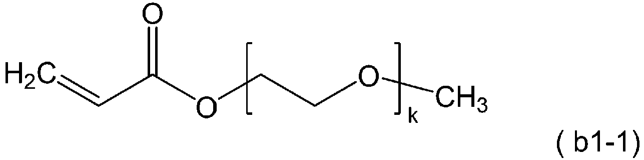 [in the formula, k represents an integer of 1 to 12].

9. The antifibrotic agent according to Claim 1,
wherein the copolymer is a copolymer represented by any of General Formulae (C-1) to (C-5), 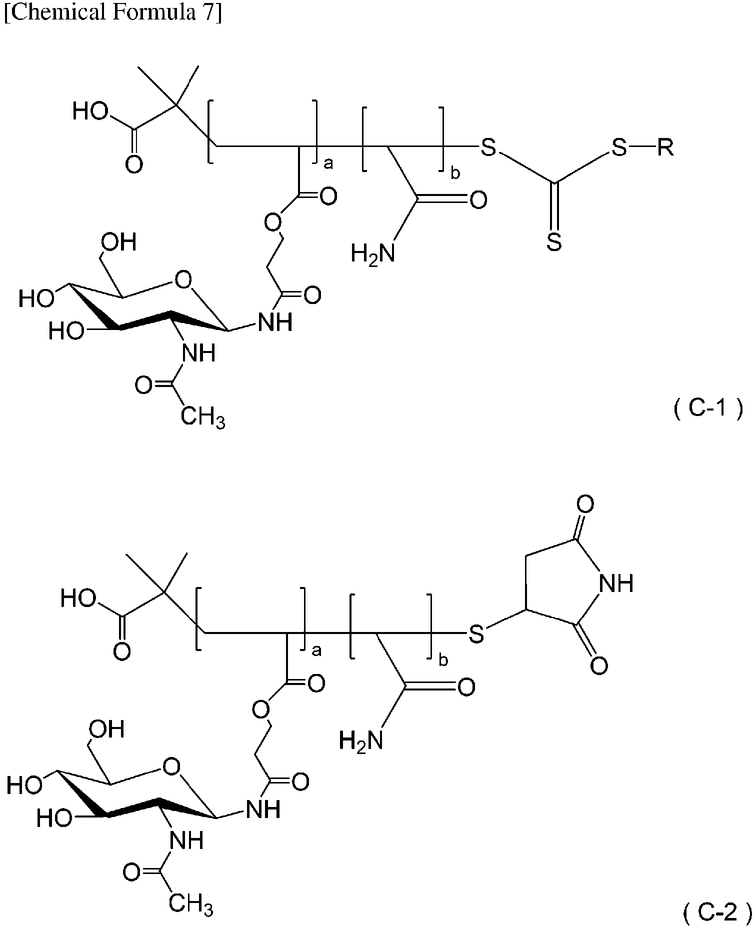 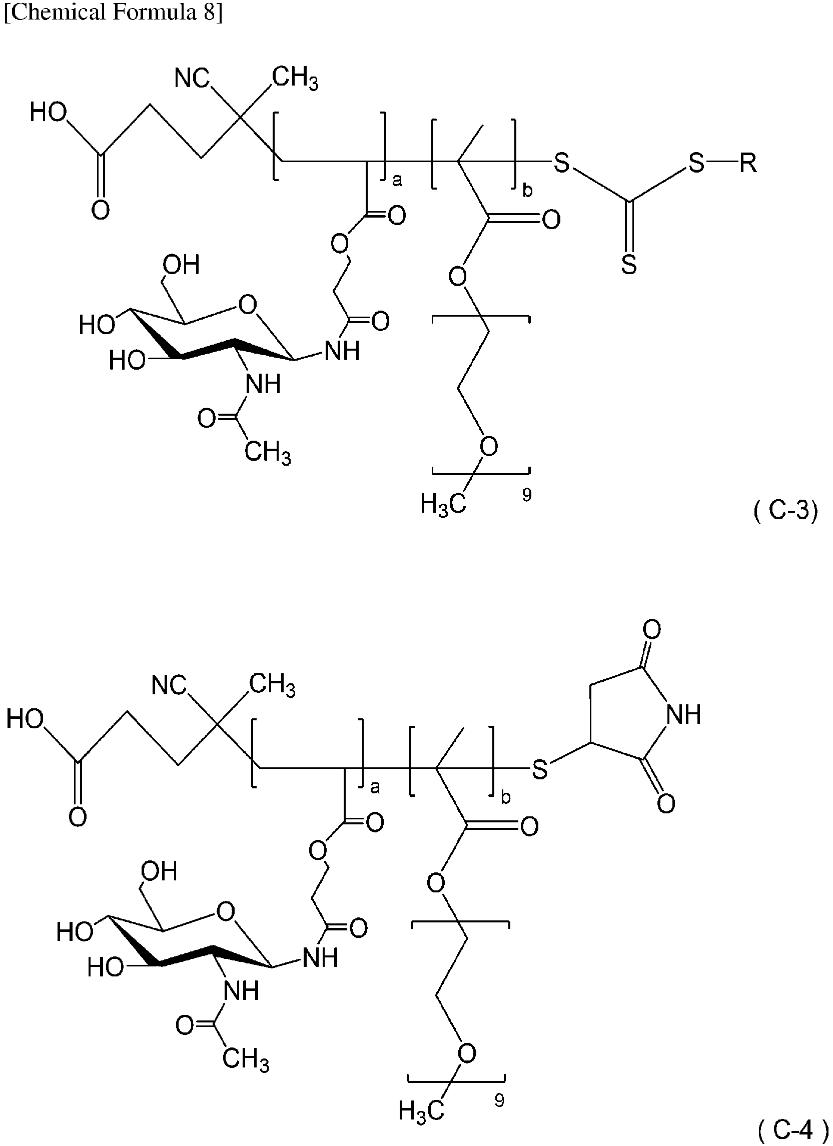 [in the formulae, R represents an alkyl group having 1 to 18 carbon atoms and a and b each independently represents an integer of 1 to 30], 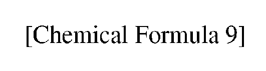 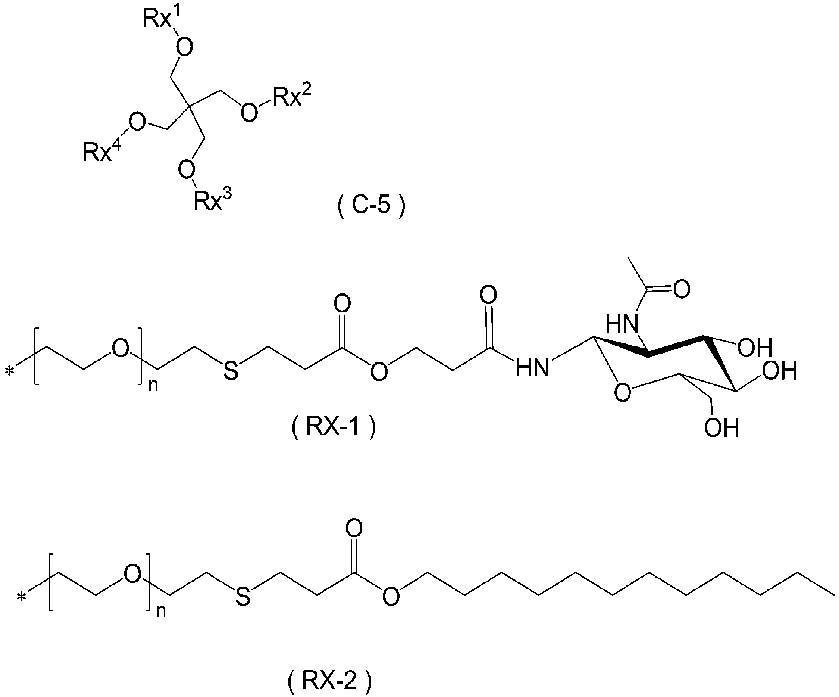 [in the formula, Rx¹ to Rx⁴ each independently represents a group represented by Formula (RX-1) or (RX-2), provided that at least one of Rx¹ to Rx⁴ is a group represented by Formula (RX-1), n represents an integer of 1 or more, and * represents a bonding site].

10. The antifibrotic agent according to any one of Claims 1 to 3,
wherein a weight-average molecular weight of the copolymer is within a range of 2,000 to 20,000.

11. The antifibrotic agent according to any one of Claims 1 to 3,
wherein a number-average molecular weight of the copolymer is within a range of 2,000 to 15,000.

12. The antifibrotic agent according to any one of Claims 1 to 3,
wherein a ratio (Mw/Mn) of a weight-average molecular weight (Mw) to a number-average molecular weight (Mn) of the copolymer is within a range of 1.0 to 1.7.

13. A pharmaceutical composition for treating fibrosis, comprising:
the antifibrotic agent according to any one of Claims 1 to 3 as an active ingredient.

14. A method of inactivating myofibroblasts, the method comprising:
binding the antifibrotic agent according to any one of Claims 1 to 3 to the myofibroblasts.
